(19) Europäisches Patentamt / European Patent Office / Office européen des brevets

(11) **EP 2 437 664 B1**

(12) **FASCICULE DE BREVET EUROPEEN**

(45) Date de publication et mention de la délivrance du brevet:
**23.11.2016 Bulletin 2016/47**

(21) Numéro de dépôt: **10737093.4**

(22) Date de dépôt: **01.06.2010**

(51) Int Cl.:
*G01R 33/563* (2006.01)   *A61B 5/02* (2006.01)
*A61B 5/026* (2006.01)   *A61B 5/0275* (2006.01)
*A61B 5/055* (2006.01)   *A61B 6/00* (2006.01)
*G01R 33/56* (2006.01)   *A61B 6/03* (2006.01)
*G06F 19/00* (2011.01)

(86) Numéro de dépôt international:
**PCT/FR2010/051068**

(87) Numéro de publication internationale:
**WO 2010/139895 (09.12.2010 Gazette 2010/49)**

(54) **PROCÉDÉ POUR ESTIMER DES PARAMÈTRES HÉMODYNAMIQUES PAR ESTIMATION CONJOINTE DES PARAMÈTRES D'UN MODÈLE GLOBAL DE PERFUSION**

VERFAHREN ZUR ABSCHÄTZUNG HÄMODYNAMISCHER PARAMETER MITTELS GEMEINSAMER ABSCHÄTZUNG DER PARAMETER EINES UMFASSENDEN PERFUSIONSMODELLS

METHOD FOR ESTIMATING HAEMODYNAMIC PARAMETERS BY JOINT ESTIMATION OF THE PARAMETERS OF A COMPREHENSIVE PERFUSION MODEL

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **05.06.2009 US 213417 P**
**09.11.2009 US 259268 P**
**30.11.2009 FR 0905759**

(43) Date de publication de la demande:
**11.04.2012 Bulletin 2012/15**

(73) Titulaire: **Olea Medical**
**13600 La Ciotat (FR)**

(72) Inventeurs:
 • **DJERIDANE, Fayçal**
 **F-13004 Marseille (FR)**
 • **PAUTOT, Fabrice**
 **F-13600 La Ciotat (FR)**

(74) Mandataire: **Brun, Philippe Alexandre Georges et al**
 **MED'INVENT CONSULTING**
 **Espace Mistral - Bât.A**
 **297 avenue du Mistral**
 **ZI ATHELIA IV**
 **13705 La Ciotat Cedex (FR)**

(56) Documents cités:
 • **SHIMONY J S ET AL: "Estimation of Cerebral Blood Flow from Dynamic Susceptibility Contrast MRI using a Tissue Model" AIP CONFERENCE PROCEEDINGS, AMERICAN INSTITUTE OF PHYSICS, NEW YORK, US LNKD-DOI:10.1063/1.2149835, vol. 803, 7 août 2005 (2005-08-07), pages 535-542, XP007915179 ISSN: 0094-243X**
 • **VONKEN E P A ET AL: "Maximum likelihood estimation of cerebral blood flow in dynamic susceptibility contrast MRI" MAGNETIC RESONANCE IN MEDICINE, ACADEMIC PRESS, DULUTH, MN, US LNKD-DOI:10.1002/(SICI)1522-2594(199902)41:2<343::AID-MRM19>3.0.CO;2-T, vol. 41, no. 2, 1 janvier 1999 (1999-01-01), pages 343-350, XP007915180 ISSN: 0740-3194**
 • **BRUNECKER ET AL: "Correcting saturation effects of the arterial input function in dynamic susceptibility contrast-enhanced MRI - a Monte Carlo simulation" MAGNETIC RESONANCE IMAGING, ELSEVIER SCIENCE, TARRYTOWN, NY, US LNKD- DOI:10.1016/J.MRI.2007.03.011, vol. 25, no. 9, 25 octobre 2007 (2007-10-25), pages 1300-1311, XP022314281 ISSN: 0730-725X**

- KIM S ET AL: "Multiphasic contrast injection for improved precision of parameter estimates in functional CT" MEDICAL PHYSICS, AIP, MELVILLE, NY, US LNKD-DOI:10.1118/1.3021138, vol. 35, no. 12, 24 novembre 2008 (2008-11-24), pages 5921-5933, XP012115882 ISSN: 0094-2405
- MILES K A ET AL: "Perfusion CT: a worthwhile enhancement?" BRITISH JOURNAL OF RADIOLOGY, BRITISH INSTITUTE OF RADIOLOGY, LONDON, GB LNKD-DOI:10.1259/BJR/13564625, vol. 76, 1 avril 2003 (2003-04-01), pages 220-231, XP002529838 ISSN: 0007-1285
- MOURIDSEN K ET AL: "Bayesian estimation of cerebral perfusion using a physiological model of microvasculature" NEUROIMAGE, ACADEMIC PRESS, ORLANDO, FL, US LNKD-DOI:10.1016/J.NEUROIMAGE.2006.06.015, vol. 33, no. 2, 1 novembre 2006 (2006-11-01), pages 570-579, XP024906828 ISSN: 1053-8119 [extrait le 2006-11-01]
- BENNER T ET AL: "Accuracy of gamma-variate fits to concentration-time curves from dynamic susceptibility-contrast enhanced MRI: Influence of time resolution, maximal signal drop and signal-to-noise" MAGNETIC RESONANCE IMAGING, ELSEVIER SCIENCE, TARRYTOWN, NY, US LNKD-DOI:10.1016/S0730-725X(96)00392-X, vol. 15, no. 3, 1 janvier 1997 (1997-01-01), pages 307-317, XP007915300 ISSN: 0730-725X [extrait le 1998-04-27]

**Description**

L'invention concerne un système et un procédé pour estimer un ou plusieurs paramètres hémodynamiques de perfusion. L'invention s'appuie notamment sur des techniques d'imagerie de Perfusion par Résonance Magnétique *(Perfusion Weighted Magnetic Resonance Imaging* (PW-MRI) en langue anglaise) ou par Tomodensitométrie *(Computed Tomography* (CT) en langue anglaise). Ces techniques permettent d'obtenir rapidement des informations précieuses sur l'hémodynamique d'organes tels que le cerveau ou le coeur. Ces informations sont particulièrement cruciales pour un praticien cherchant à établir un diagnostic et à prendre une décision thérapeutique dans le traitement en urgence de pathologies telles que les accidents vasculaires cérébraux.

Pour mettre en oeuvre de telles techniques, on utilise un appareil d'imagerie par Résonance Magnétique Nucléaire ou par Tomodensitométrie. Celui-ci délivre une pluralité de séquences d'images numériques d'une partie du corps, notamment du cerveau. Ledit appareil applique pour cela une combinaison d'ondes électromagnétiques à haute fréquence sur la partie du corps considérée et mesure le signal réémis par certains atomes. L'appareil permet ainsi de déterminer la composition chimique et donc la nature des tissus biologiques en chaque point (ou voxel) du volume imagé.

Des séquences d'images sont analysées au moyen d'une unité de traitement dédiée. Cette unité de traitement délivre in fine à un praticien, une estimation des paramètres hémodynamiques à partir des images de perfusion, au moyen d'une interface homme-machine adaptée. Le praticien peut ainsi réaliser un diagnostic et décider de l'action thérapeutique qu'il jugera adéquate.

Des images de perfusion par Résonance Magnétique Nucléaire ou par Tomodensitométrie sont obtenues en injectant un agent de contraste (par exemple un sel de gadolinium pour l'Imagerie par Résonance Magnétique) par voie intraveineuse et en enregistrant son bol au cours du temps au niveau de chaque voxel de l'image. Par souci de concision, nous omettrons les indices $x, y, z$ pour identifier des voxels. Par exemple, au lieu de noter $S_{x,y,z}(t)$ le signal pour un voxel de coordonnées $x, y, z$, nous le noterons simplement $S(t)$. Il est entendu que les opérations et les calculs décrits dans la suite sont généralement effectués pour chaque voxel d'intérêt, de sorte à obtenir au final des images ou des cartes représentatives des paramètres hémodynamiques que l'on cherche à estimer.

Un modèle standard permet de relier l'intensité des signaux $S(t)$ mesurée au cours du temps $t$, à la concentration $C(t)$ dudit agent de contraste.

Par exemple, en Tomodensitométrie de Perfusion, le signal pour chaque voxel est directement proportionnel à la concentration : $S(t) = \alpha.C(t)$, $\alpha$ étant une constante non nulle.

En Imagerie de Perfusion par Résonance Magnétique Nucléaire, il existe une relation exponentielle $S(t) = S_0.e^{-k.TE.C(t)}$ où $S_0$ est l'intensité moyenne du signal avant l'arrivée de l'agent de contraste, *TE* est le temps d'écho *(echo time* en langue anglaise) et $k$ est une constante dépendant de la relation entre la susceptibilité paramagnétique et la concentration de l'agent de contraste dans le tissu. La valeur de la constante $k$ pour chaque voxel étant inconnue, celle-ci est fixée à une valeur arbitraire pour tous les voxels d'intérêt. On obtient ainsi des estimations relatives et non pas absolues. Lesdites informations relatives restent pertinentes puisque l'on est intéressé principalement par la variation de ces valeurs relatives dans l'espace.

La concentration $C(t)$ peut alors être exprimée elle-même par le modèle de convolution standard de perfusion $C(t) = BF \cdot C_a(t) \otimes R(t)$ où $C_a(t)$ est la concentration de l'agent de contraste dans l'artère alimentant le volume de tissu dans un voxel (Fonction d'Entrée Artérielle ou *Arterial Input Function (AIF)* en langue anglaise), *BF* est le Flux Sanguin dans le volume de tissu *(Blood Flow* en langue anglaise), $R(t)$ est la Fonction de Répartition Complémentaire du temps de transit dans le volume de tissu *(residue function* en langue anglaise) et $\otimes$ désigne le produit de convolution.

Partant d'un signal expérimental $S(t)$, des procédés connus consistent à estimer tout d'abord $S_0$ en prenant, par exemple, sa moyenne avant que l'agent de contraste n'arrive. On obtient ainsi une estimation de la concentration de l'agent de contraste $C(t)$ grâce à la relation

$$C(t) = -\frac{1}{k \cdot TE} \ln\left[\frac{S(t)}{S_0}\right].$$

Connaissant $C(t)$ et la fonction d'entrée artérielle $C_a(t)$, on peut alors obtenir par déconvolution numérique la fonction $BF.R(t)$ sous le modèle standard de perfusion.

Par suite, on peut obtenir une estimation du paramètre *BF* puisque $R(0)=1$ par définition. On obtient en outre une estimation du Temps de Transit Moyen dans le tissu *(Mean Transit Time* (*MTT*) en langue anglaise), puisque

$$MTT = \int_0^{+\infty} R(t)\,dt.$$ On peut obtenir également une estimation du volume sanguin dans le tissu *(Blood Volume* (BV) en langue anglaise) par la relation $BV = BF.MTT$.

Selon l'état de la technique, il ne semble pas possible de concevoir une unité de traitement apte à mettre en oeuvre un procédé de déconvolution du modèle standard de perfusion $C(t) = BF \cdot C_a(t) \otimes R(t)$ sans devoir fournir une fonction

d'entrée artérielle $C_a(t)$. En effet ledit modèle est exprimé sous la forme d'une seule équation et il existe deux inconnues.

Des techniques ont donc été envisagées, par exemple celles décrites dans les documents « Maximum Likelihood Estimation of Cerebral Blood Flow in Dynamic Susceptibiliy Contrat MRI - Vonken et al. », « Bayesian estimation of cerebral perfusion using a physiological model of micro vasculature - Mouridsen et al. », « Multiphasic contrast injection for improved precision of parameter estimated in functional CT - Kim et al. » ou encore « Correcting saturation effects of the arterial input function in Dynamic Susceptibility Contrast-enhanced MRI - Brunecker et al. », pour fixer une ou plusieurs Fonctions d'Entrée Artérielles $C_a(t)$ et rendre ainsi possible la déconvolution de courbes de concentration $C(t)$ afin d'estimer les paramètres hémodynamiques.

Pour estimer des paramètres hémodynamiques d'un organe tel que le cerveau humain, selon une première technique, une fonction d'entrée artérielle globale est choisie manuellement par un praticien. Le choix peut porter par exemple sur l'artère sylvienne contralatérale à l'hémisphère pathologique dans le cas de l'imagerie de perfusion du cerveau. On peut obtenir en variante une fonction d'entrée artérielle globale au moyen de mesures supplémentaires, par exemple optiques.

Si elle permet d'obtenir des signaux avec hauts rapports signal sur bruit, cette technique induit néanmoins de nombreux inconvénients. Tout d'abord, elle nécessite une intervention humaine et/ou des mesures supplémentaires. Ceci peut être très pénalisant en situation d'urgence clinique. En outre, les procédures et les résultats finaux sont difficilement reproductibles. Ensuite et surtout, la fonction d'entrée artérielle globale ne correspond pas aux fonctions d'entrée artérielle locales pour les tissus d'intérêt. Elle en diffère en termes de délai car les fonctions d'entrée artérielle locales sont le plus souvent en retard par rapport à la fonction d'entrée artérielle globale prise en amont. Elle en diffère en outre en termes de dispersion car la propagation de l'agent de contraste est plus lente en aval qu'en amont. Or il est connu que de tels phénomènes ont une influence considérable sur les estimations des paramètres hémodynamiques. En particulier, selon cette première technique, on n'obtient pas une estimation du véritable Temps de Transit Moyen *MTT* entre la fonction d'entrée artérielle locale et la fonction de sortie veineuse. On obtient seulement un Temps de Transit Moyen entre la fonction d'entrée artérielle globale et la fonction de sortie veineuse. Pour tenter de quantifier ces discordances par rapport au modèle standard de perfusion, certains ont introduit de nouveaux paramètres descriptifs, tels qu'un paramètre $TMAX = \arg\max_{t} R(t)$, quand bien même lesdits paramètres n'interviennent pas dans le modèle de perfusion standard. D'autres méthodes tendent à minimiser l'influence de ces discordances sur l'estimation des paramètres hémodynamiques mais introduisent de nouvelles inconnues dans le problème global.

Selon une deuxième technique, une fonction d'entrée artérielle globale est obtenue automatiquement à partir d'images de perfusion au moyen de techniques de traitement du signal telles que le Partitionnement de Données (*Clustering* en langue anglaise) ou l'Analyse en Composantes Indépendantes *(Independent Component Analysis (ICA)* en langue anglaise). Cette deuxième technique permet de s'affranchir de l'intervention humaine. Toutefois cette approche ne résout pas les problèmes de délai et de dispersion inhérents aux fonctions d'entrée artérielle globales.

Selon une troisième approche, des fonctions d'entrée artérielles locales sont produites automatiquement à partir d'images de perfusion à l'aide de techniques de traitement du signal et de critères de sélection. On recherche par exemple la « meilleure » fonction dans un voisinage immédiat du voxel courant pour lequel on souhaite estimer les paramètres hémodynamiques. L'objet de cette troisième technique est d'obtenir au final des estimations moins biaisées et plus précises. On s'affranchit, du moins en partie, des problèmes de délai et de dispersion. Cependant, rien ne garantit a priori et a posteriori que les fonctions d'entrée artérielle locales ainsi obtenues sont des approximations pertinentes de la « véritable fonction locale » pour le voxel d'intérêt. Par exemple, cette « véritable » fonction pourrait ne pas se situer dans le voisinage considéré si celui-ci est trop petit. Ladite « véritable » fonction pourrait également être confondue avec une autre fonction d'entrée artérielle, si le voisinage considéré est trop grand. Par conséquent, même si les résultats peuvent être meilleurs qu'avec une fonction d'entrée artérielle globale, les incertitudes pesant sur les fonctions d'entrée artérielles locales (et a fortiori sur les paramètres hémodynamiques) demeurent en grande partie.

Quelle que soit la technique jusqu'alors employée, la déconvolution de la courbe de concentration par la fonction d'entrée artérielle, que celle-ci soit globale ou locale (et par conséquent l'estimation des paramètres hémodynamiques) est effectuée en utilisant, par exemple, des méthodes de déconvolution non-paramétriques telles que la Décomposition en Valeurs Singulières (*Singular Value Decomposition* en langue anglaise), la Déconvolution de Hunt dans le domaine fréquentiel, la régularisation de Tikhonov, etc.

Des méthodes utilisant des modèles physiologiques paramétriques et semi-paramétriques $C_a(t, \Theta_a)$ pour la fonction d'entrée artérielle et $R(t, \Theta_R)$ pour la fonction de répartition complémentaire du temps de transit dans le tissu, ont été toutefois proposées dans la littérature. L'utilisation connue de tels modèles n'a pas pour objet de supplanter l'opération de déconvolution mais de tenter d'améliorer certains résultats obtenus par les méthodes non paramétriques telles que décrites précédemment.

Par exemple, un modèle physiologique paramétrique $C_a(t, \Theta_a)$ de la fonction d'entrée artérielle locale, modèle « mono-Gamma » à une composante et quatre paramètres

$$
\begin{cases}
C_a\left(t,\Theta_a\right) = \dfrac{a\left(t-t_0\right)^{\alpha_0-1} e^{-\left(t-t_0\right)/\beta_0}}{\beta_0^{\alpha_0}\Gamma\left(\alpha_0\right)} \\[2mm]
\Theta_a = \left(a,\alpha_0,\beta_0,t_0\right) \\[2mm]
\Gamma(\ ) \text{ fonction Gamma d'Euler}
\end{cases}
$$

a été introduit pour :

- synthétiser des données de simulation pour l'étude et la validation des méthodes de déconvolution non-paramétriques ;
- réduire le bruit de mesure et lisser des données en remplaçant la ou les fonctions d'entrée artérielle expérimentales par un ajustement de ces modèles lisses auxdites courbes avant d'effectuer la déconvolution numérique et l'estimation des paramètres ;
- pallier les problèmes liés à l'échantillonnage temporel des fonctions d'entrée artérielle en sur-échantillonnant artificiellement un ajustement de ces modèles auxdites courbes avant d'effectuer la déconvolution numérique ;
- pallier les problèmes dus au phénomène de recirculation de l'agent de contraste dans le voxel d'intérêt, lorsqu'elle recouvre temporellement le premier passage de l'agent de contraste.

De même, des modèles paramétriques ou semi-paramétriques $R(t,\Theta_R)$ de la fonction de répartition complémentaire du temps de transit dans le tissu, ont été proposés, parmi lesquels nous pouvons citer :

- le modèle paramétrique « créneau » (*box-shaped*) :

$$
\begin{cases}
R\left(t,\Theta_R\right) = \chi_{[0.MTT]}(t) \quad t \geq 0 \\[2mm]
\Theta_R = MTT \\[2mm]
\chi_E(\ ) \text{ fonction indicatrice}
\end{cases}
$$

- le modèle paramétrique « triangle » :

$$
\begin{cases}
R\left(t,\Theta_R\right) = \left(1 - \dfrac{t}{2 \cdot MTT}\right)\chi_{[0.2 \cdot MTT]}(t) \quad t \geq 0 \\[2mm]
\Theta_R = MTT \\[2mm]
\chi_E(\ ) \text{ fonction indicatrice}
\end{cases}
$$

- le modèle paramétrique « monoexponentiel » (monoexponential) :

$$
\begin{cases}
R\left(t,\Theta_R\right) = e^{-\frac{t}{MTT}} \quad t \geq 0 \\[2mm]
\Theta_R = MTT
\end{cases}
$$

- le modèle paramétrique « Gamma Intégral » à deux paramètres :

$$\begin{cases} R(t, \Theta_R) = \int\limits_{t}^{+\infty} h(\tau, \alpha, \beta) d\tau \quad t \geq 0 \\[2mm] h(\tau, \alpha, \beta) = \dfrac{t^{\alpha-1} e^{-t/\beta}}{\beta^{\alpha} \Gamma(\alpha)} \quad \alpha > 0, \beta > 0 \\[2mm] \Theta_R = (\alpha, \beta) \\[2mm] \Gamma(\ ) \text{ fonction Gamma d'Euler} \end{cases}$$

Ces modèles ont été introduits pour :

- synthétiser des données de simulation pour l'étude et la validation des méthodes de déconvolution non-paramétriques ; c'est par exemple le cas pour les modèles « créneau » et « triangle » qui représentent des cas extrêmes qui ne peuvent pas être rencontrés dans la nature ;
- obtenir, réciproquement par ajustement de ces modèles aux données expérimentales, des fonctions de répartition complémentaire du temps de transit dans le tissu $R(t)$ plus « physiologiques » que celles obtenues par les méthodes non-paramétriques (en particulier, des fonctions positives, décroissantes et valant un à l'origine, telles que définies par le modèle standard de perfusion) ;
- obtenir analytiquement d'autres fonctions et paramètres d'intérêt dépendant de la fonction de répartition complémentaire, telle que la fonction de densité de probabilité $h(t)$ du temps de transit dans le tissu (réponse impulsionnelle)

donnée par $h(t) = \delta(t) - \dfrac{dR(t)}{dt}$ où $\delta(t)$ est la fonction généralisée de Dirac.

L'utilisation de tels modèles a permis d'améliorer peu ou prou certaines estimations de paramètres. Toutefois, les inconvénients inhérents au fait de fixer la fonction d'entrée artérielle, que celle-ci soit globale ou locale, restent présents.

L'invention permet de répondre à l'ensemble des inconvénients soulevés par les solutions connues. Elle offre des procédures rapides, objectives et reproductibles. L'invention ne porte pas sur une méthode de diagnostic en tant que telle car un procédé selon l'invention n'englobe pas toutes les étapes permettant un tel diagnostic. En particulier, l'invention ne concerne pas des phases d'investigation permettant un recueil de données réalisées par exemple au moyen d'un appareil d'imagerie par Résonance Magnétique Nucléaire. L'invention ne concerne pas en outre d'étapes d'interaction avec un corps humain ou animal permettant à un praticien de mettre en oeuvre un diagnostic.

Toutefois, les avantages apportés par l'invention sont nombreux et significatifs. L'invention peut se révéler particulièrement appréciable en situation d'urgence clinique, offrant ainsi à un praticien une aide pour parfaire un diagnostic et prendre une décision thérapeutique adéquate.

La mise en oeuvre de l'invention permet en outre à un chercheur de progresser dans la compréhension physiologique d'un organe d'un humain ou d'un animal.

Parmi les nombreux avantages, nous pouvons mentionner:

- la suppression de toute intervention humaine et/ou de mesures expérimentales additionnelles pour tenter de choisir manuellement ou automatiquement une fonction d'entrée artérielle globale ou des fonctions d'entrée artérielle locales ;
- obtenir des estimations des paramètres hémodynamiques d'intérêt moins biaisées et plus précises en s'affranchissant de problèmes de déconvolution « mal-posés » et numériquement instables ainsi que par construction, des problèmes de délai et de dispersion inhérents aux fonctions d'entrée artérielle globales ou locales ;

- fournir optionnellement des estimations des fonctions d'entrée artérielle locales ainsi que des fonctions de répartition complémentaire pour chaque voxel de tissu afin, par exemple, de comparer et de valider l'apport de l'invention par rapport à l'état de l'art ;
- fournir optionnellement des intervalles de confiance sur l'estimation de chacun des paramètres et même des paris sur lesdits intervalles de confiance ;
- fournir optionnellement une mesure quantitative et objective de l'adéquation du modèle de perfusion global aux données expérimentales et, par conséquent, permettre la comparaison et la sélection de modèles les plus adéquats.

A cette fin, il est prévu un procédé tel que défini par la revendication 1 pour estimer un ou plusieurs paramètres hémodynamiques de perfusion d'un volume élémentaire - dit voxel - d'un organe, ledit procédé étant mis en oeuvre par une unité de traitement d'un système d'analyse d'imagerie de perfusion, et comportant une étape pour estimer un ou plusieurs paramètres hémodynamiques à partir de signaux de perfusion $S(t)$. Selon l'invention, l'étape pour estimer le ou lesdits paramètres hémodynamiques consiste en l'évaluation d'une distribution marginale a posteriori pour le ou lesdits paramètres hémodynamiques par l'assignation préalable d'une distribution conjointe a priori des paramètres $\Theta$ d'un modèle global de perfusion comportant :

- une première relation entre le signal de perfusion $S(t)$ et une concentration $C(t)$ d'un agent de contraste circulant dans ledit voxel au cours du temps t *;*
- une deuxième relation entre la concentration $C(t)$ d'un agent de contraste, le flux sanguin $BF$, un modèle paramétrique ou semi-paramétrique $C_a(t,\Theta_a)$ d'une fonction d'entrée artérielle et un modèle paramétrique ou semi-paramétrique $R(t,\Theta_R)$ d'une fonction de répartition complémentaire du temps de transit dans le voxel, $\Theta_a$ et $\Theta_R$ étant respectivement les paramètres desdits modèles $C_a(t,\Theta_a)$ et $R(t,\Theta_R)$ ;
- une troisième relation $\Psi(\Theta_a) = 0$ entre les paramètres $\Theta_a$ du modèle de la fonction d'entrée artérielle $C_a(t,\Theta_a)$ consistant à exprimer l'un desdits paramètres en fonction des autres, ladite troisième relation étant identique pour tout voxel d'intérêt et s'exprimant comme: $\int_0^{+\infty} C_a(t,\Theta_a)\,dt = C_0$ où $C_0$ est une constante arbitraire non 0 nulle, identique pour tout voxel d'intérêt.

Selon un premier exemple d'application de l'invention :

- un signal de perfusion $S(t)$ peut être préalablement obtenu à partir d'images numériques délivrées par un appareil d'imagerie de perfusion par résonance magnétique ;
- la première relation d'un modèle global de perfusion peut être exprimée par $S(t)=S_0 e^{-k \cdot TE \cdot C(t)}$ où $S_0$ est l'intensité moyenne du signal avant l'arrivée de l'agent de contraste dans le voxel, $TE$ est un temps d'écho *et k* une constante non nulle ;
- la deuxième relation dudit modèle peut être exprimée par $C(t)=\eta \cdot BF \cdot C_a(t,\Theta_a) \otimes R(t,\Theta_R)$ où $\otimes$ désigne le produit de convolution, $\eta$ est une constante non nulle et $BF$ est le flux sanguin circulant dans le voxel considéré.

Selon un deuxième exemple d'application de l'invention :

- un signal de perfusion $S(t)$ peut être préalablement obtenu à partir d'images numériques délivrées par un appareil d'imagerie de perfusion par tomodensitométrie ;
- la première relation d'un modèle global de perfusion peut être une relation de proportionnalité $S(t)=\alpha.C(t)$ où $\alpha$ est une constante non nulle;
- la deuxième relation dudit modèle peut s'exprimer par $C(t)=\eta \cdot BF \cdot C_a(t,\Theta_a) \otimes R(t,\Theta_R)$ où $\otimes$ désigne le produit de convolution, $\eta$ est une constante non nulle et $BF$ est le flux sanguin circulant dans le voxel considéré.

Selon un premier mode de réalisation, un procédé conforme à l'invention peut être mis en oeuvre par itérations successives pour une pluralité de voxels considérés.

A titre d'exemple, l'invention prévoit que l'estimation conjointe des paramètres $\Theta$ du modèle global de perfusion mise en oeuvre par l'unité de traitement peut être réalisée au moyen de la méthode de Bayes ou des méthodes du Maximum de Vraisemblance ou encore des méthodes des Moindres Carrés non linéaires.

Selon un mode de réalisation préféré, le procédé peut comporter une étape pour quantifier l'adéquation du modèle global de perfusion à des données de perfusion $D=[S(t_1),...,S(t_N)]$ expérimentales par un calcul d'une probabilité $p(D|M) = \int_\Theta p(\Theta|M)\,p(D|\Theta,M)\,d\Theta$ de ces données sachant ledit modèle.

Il peut être en outre prévu que le procédé selon l'invention puisse comporter une étape préalable pour choisir un modèle global de perfusion parmi une pluralité.

Ainsi il est possible qu'un tel procédé puisse être mis en oeuvre itérativement par l'unité de traitement pour chaque modèle global de perfusion connu de ladite unité de traitement. Selon le mode de réalisation préféré précédemment décrit, il peut être alors prévu que soient délivrés les paramètres estimés selon le modèle de perfusion dont la probabilité sachant les données est la plus grande.

Outre une estimation d'un ou plusieurs paramètres hémodynamiques, l'invention prévoit que le procédé puisse comporter une étape pour calculer une information complémentaire sous la forme :

- d'une fonction d'entrée artérielle estimée $C_a(t,\widehat{\Theta_a})$ ;

- d'une fonction de répartition complémentaire estimée $R(t,\widehat{\Theta_R})$ ;

- d'un intervalle de confiance associé à un paramètre du modèle global de perfusion ;

- ou encore d'un taux de pari associé à un paramètre du modèle global de perfusion.

L'invention prévoit un mode de réalisation du procédé pour qu'il puisse comporter une étape pour délivrer des paramètres hémodynamiques estimés, voire toutes autres informations complémentaires qui leur seraient associées, à une interface homme-machine apte à restituer à un utilisateur lesdits paramètres estimés.

L'invention concerne en outre une unité de traitement telle que définie par la revendication 18, comportant des moyens de mémorisation, des moyens pour communiquer avec le monde extérieur et des moyens de traitement, de sorte que :

- les moyens pour communiquer sont aptes à recevoir du monde extérieur un signal $S(t)$ obtenu par imagerie de perfusion ;
- les moyens de mémorisation comportent un modèle global de perfusion, ledit modèle comportant :

  - une première relation entre le signal de perfusion $S(t)$ et une concentration $C(t)$ d'un agent de contraste circulant dans ledit voxel au cours du *temps t* ;
  - une deuxième relation entre la concentration $C(t)$, le flux sanguin $BF$, un modèle paramétrique ou semi-paramétrique $C_a(t,\Theta_a)$ d'une fonction d'entrée artérielle et un modèle paramétrique ou semi-paramétrique $R(t,\Theta_R)$ d'une fonction de répartition complémentaire du temps de transit dans le voxel, $\Theta_a$ et $\Theta_R$ étant respectivement les paramètres desdits modèles ;
  - une troisième relation $\Psi(\Theta_a)=0$ entre les paramètres $\Theta_a$ du modèle de la fonction d'entrée artérielle $C_a(t,\Theta_a)$ consistant à exprimer l'un desdits paramètres en fonction des autres, ladite troisième relation étant identique pour tout voxel d'intérêt et s'exprimant comme: $\int_0^{+\infty} C_a(t,\Theta_a)\,dt = C_0$ où $C_0$ est une constante arbitraire non 0 nulle, identique pour tout voxel d'intérêt;

- les moyens de traitement sont adaptés pour mettre en oeuvre un procédé pour estimer des paramètres hémodynamiques de perfusion conforme à l'invention.

L'invention concerne en variante une unité de traitement telle que définie par la revendication 19, dont les moyens pour communiquer sont aptes à recevoir du monde extérieur des images numériques de perfusion à partir desquelles les moyens de traitement déterminent un signal de perfusion $S(t)$ et mettent en oeuvre un procédé pour estimer un ou plusieurs paramètres hémodynamiques de perfusion conforme à l'invention.

L'invention prévoit en outre que les moyens pour communiquer d'une unité de traitement conforme à l'invention, peuvent délivrer un paramètre estimé selon un format approprié à une interface homme-machine apte à restituer à un utilisateur ledit paramètre estimé.

L'invention concerne également tout système d'analyse d'imagerie de perfusion tel que défini par la revendication 21, comportant une unité de traitement telle que décrite précédemment et une interface homme-machine apte à restituer à un utilisateur un ou plusieurs paramètres estimés selon un procédé conforme à l'invention et mis en oeuvre par ladite unité de traitement.

D'autres caractéristiques et avantages apparaîtront plus clairement à la lecture de la description qui suit et à l'examen des figures qui l'accompagnent parmi lesquelles :

- les figures 1 et 2 présentent deux variantes de réalisation d'un système d'analyse d'imagerie de perfusion ;
- Les figures 3 et 4 présentent respectivement une image de perfusion, obtenue par un appareil d'imagerie par Résonance Magnétique Nucléaire, d'une tranche d'un cerveau humain avant l'injection d'un agent de contraste et pendant la circulation de celui-ci dans les tissus dudit cerveau ;

- les figures 5a et 5b présentent un signal de perfusion *S(t)* par Résonance Magnétique Nucléaire typique relatif à un voxel d'un cerveau humain ;
- la figure 6 présente une courbe de concentration *C(t)* typique d'un agent de contraste circulant au sein d'un voxel d'un cerveau humain;
- la figure 7 présente une fonction d'entrée artérielle $C_a(t)$ typique ;
- la figure 8 présente un procédé conforme à l'invention ;
- les figures 9 à 14 présentent respectivement une carte relative à un paramètre hémodynamique estimé conformément à l'invention ;
- les figures 15 et 16 concernent respectivement des écart-types des flux sanguins et du temps de transit moyen estimés conformément à l'invention ;
- la figure 17 concerne une carte relative à une probabilité qu'un flux sanguin cérébral soit dans un intervalle de confiance.

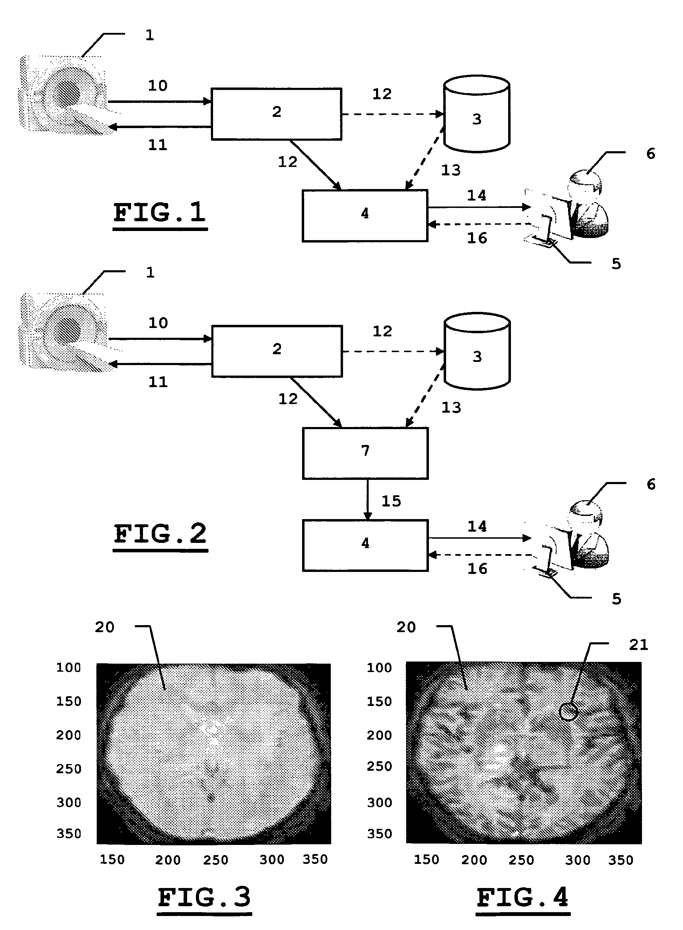

La figure 1 permet de présenter un système d'analyse d'images de perfusion. Un appareil 1 d'imagerie par Résonance Magnétique Nucléaire ou par Tomodensitométrie est commandé à l'aide d'une console 2. Un utilisateur peut ainsi choisir des paramètres 11 pour piloter l'appareil 1. A partir d'informations 10 produites par l'appareil 1, on obtient une pluralité de séquences d'images numériques 12 d'une partie d'un corps d'un humain ou d'un animal. A titre d'exemple préféré, nous illustrerons les solutions issues de l'art antérieur ainsi que l'invention à l'aide d'images numériques issues de l'observation d'un cerveau humain. D'autres organes pourraient aussi être considérés.

Les séquences d'images 12 peuvent optionnellement être stockées au sein d'un serveur 3 et constituer un dossier médical 13 d'un patient. Un tel dossier 13 peut comprendre des images de différents types, telles que des images de perfusion ou de diffusion. Les séquences d'images 12 sont analysées au moyen d'une unité de traitement 4 dédiée. Ladite unité de traitement comporte des moyens pour communiquer avec le monde extérieur pour recueillir les images. Lesdits moyens pour communiquer permettent en outre à l'unité de traitement de délivrer in fine à un praticien 6 ou à un chercheur, une estimation des paramètres hémodynamiques à partir des images de perfusion 12, au moyen d'une interface homme-machine adaptée 5. L'utilisateur 6 du système d'analyse peut ainsi confirmer ou infirmer un diagnostic, décider une action thérapeutique qu'il jugera adéquate, approfondir des travaux de recherche... Optionnellement, cet utilisateur peut paramétrer le fonctionnement de l'unité de traitement 4 au moyen de paramètres 16. Par exemple, il peut ainsi définir des seuils d'affichage ou choisir les paramètres estimés qu'il souhaite visualiser.

La figure 2 illustre une variante de réalisation d'un système d'analyse pour lequel une unité de prétraitement 7 analyse des séquences d'images 12 pour en déduire des signaux de perfusion 15 par voxel. L'unité de traitement 4 chargée d'estimer les paramètres hémodynamiques 14 est ainsi déchargée de cette action et met en oeuvre un procédé d'estimation à partir de signaux de perfusion 15 réceptionnés par ses moyens pour communiquer avec le monde extérieur.

La figure 3 illustre un exemple d'image typique 12 d'une tranche de 5 millimètres d'épaisseur d'un cerveau humain. Cette image est obtenue par Résonance Magnétique Nucléaire. A l'aide de cette technique, on peut obtenir, pour chaque tranche, une matrice de 128 x 128 voxels dont les dimensions sont de 1,5 x 1,5 x 5 millimètres. A l'aide d'une interpolation bilinéaire on peut produire une image à plat de 458 x 458 pixels telle que l'image 20.

La figure 4 illustre une image 20 similaire à celle présentée en liaison avec la figure 3. Toutefois cette image est obtenue après une injection d'un agent de contraste. Cette image est un exemple d'image de perfusion typique d'un cerveau. Les artères apparaissent ainsi clairement contrairement à la même image décrite en figure 3. Selon des techniques connues, il est possible de choisir une ou plusieurs fonctions d'entrée artérielle 21 dans l'hémisphère contralatérale à l'hémisphère pathologique pour estimer des paramètres hémodynamiques.

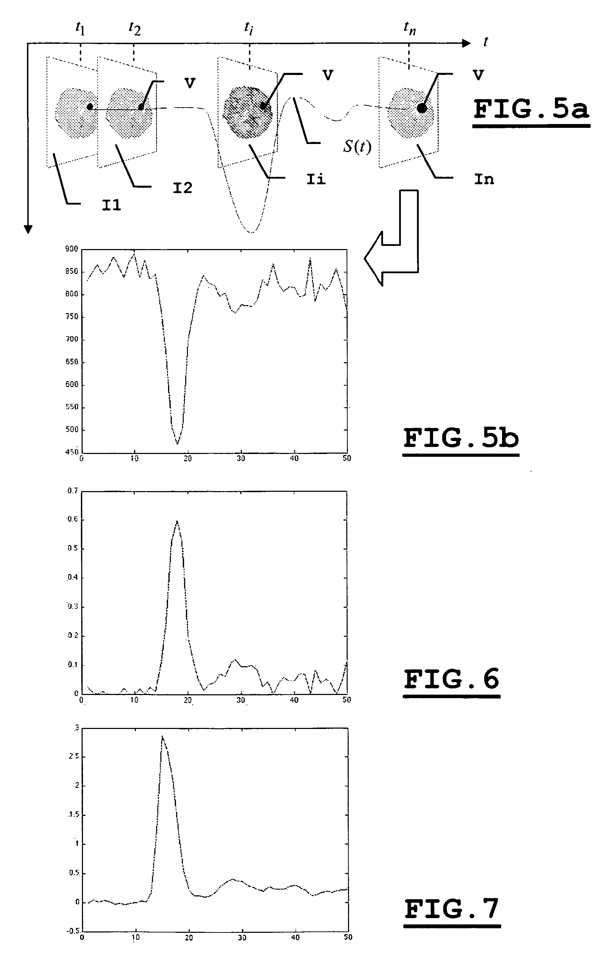

La figure 5b permet d'illustrer un exemple de signal de perfusion *S(t)* par Résonance Magnétique Nucléaire tel que les signaux 15 délivrés par l'unité de prétraitement 7 décrite en liaison avec la figure 2. Le signal de perfusion est ainsi représentatif de l'évolution d'un voxel au cours du temps t à la suite d'une injection d'un agent de contraste. A titre d'exemple, la figure 5b décrit un tel signal sur une durée de 50 secondes. L'axe des ordonnées décrit l'intensité du signal dont l'unité est arbitraire. Pour obtenir un tel signal, l'unité de traitement 4 selon la figure 1 (ou en variante l'unité de prétraitement 7 selon la figure 2), analyse une séquence de n images de perfusion par Résonance Magnétique Nucléaire I1, I2, ..., Ii, ..., In à des instants $t_1, t_2, ..., t_i, ..., t_n$ comme le décrit, à titre d'exemple, la figure 5a. Pour un voxel donné, par exemple pour le voxel V, on détermine un signal de perfusion *S(t)* représentatif de l'évolution du voxel au cours du temps *t* à la suite d'une injection d'un agent de contraste.

La figure 6 présente une courbe de concentration déduite d'un signal de perfusion tel que celui-ci décrit en figure 5b. Comme déjà évoqué précédemment, il existe une relation entre un signal de perfusion et une courbe de concentration associée. Ainsi en Imagerie de Perfusion par Résonance Magnétique Nucléaire, il existe une relation exponentielle $S(t) = S_0 . e^{-k.TE.C(t)}$ où $S_0$ est l'intensité moyenne du signal avant l'arrivée de l'agent de contraste, *TE* est le temps d'écho (*echo time*) et *k* est une constante dépendant de la relation entre la susceptibilité paramagnétique et la concentration de l'agent de contraste dans le tissu. La valeur de la constante *k* pour chaque voxel étant inconnue, celle-ci est fixée à une valeur arbitraire pour tous les voxels d'intérêt.

La figure 6 permet ainsi de visualiser au cours du temps, l'évolution de la concentration d'un agent de contraste au sein d'un voxel. On note un pic de forte amplitude lors du premier passage (*first pass,* en langue anglaise) de l'agent de contraste dans le voxel suivi de pics d'amplitudes plus faibles lié à un phénomène de recirculation (*second pass,* en langue anglaise) dudit agent de contraste.

La figure 7 illustre quant à elle, une fonction d'entrée artérielle typique $C_a(t)$ représentative de la circulation d'un agent de contraste au sein d'un voxel artériel tel que le voxel 21 présenté en liaison avec la figure 4. La figure 7 permet de constater notamment que le phénomène de recirculation après un premier passage de l'agent de contraste est très faible.

Pour mettre en oeuvre l'invention, on introduit un modèle physiologique paramétrique ou semi-paramétrique $C_a(t,\Theta_a)$ d'une fonction d'entrée artérielle. Un tel modèle est stocké par des moyens de mémorisation ou programmé dans l'unité de traitement 4 d'un système d'analyse tel que décrit en liaison avec les figures 1 et 2.

Selon un mode de réalisation préféré de l'invention, ce modèle est un modèle dit « tri-Gamma » à douze paramètres défini par :

$$
\begin{cases}
C_a\left(t,\Theta_a\right) = \dfrac{a\left(t-t_0\right)^{\alpha_0-1} e^{-\left(t-t_0\right)/\beta_0}}{\beta_0^{\alpha_0}\Gamma(\alpha_0)} + \dfrac{b\left(t-t_1\right)^{\alpha_1-1} e^{-\left(t-t_1\right)/\beta_1}}{\beta_1^{\alpha_1}\Gamma(\alpha_1)} + \dfrac{c\left(t-t_2\right)^{\alpha_2-1} e^{-\left(t-t_2\right)/\beta_2}}{\beta_2^{\alpha_2}\Gamma(\alpha_2)} \\[4mm]
\Theta_a = \left(a,b,c,\alpha_0,\beta_0,t_0,\alpha_1,\beta_1,t_1,\alpha_2,\beta_2,t_2\right) \\[2mm]
\Gamma(\ ) : \text{fonction Gamma d'Euler}
\end{cases}
$$

En outre, on introduit un modèle physiologique paramétrique ou semi-paramétrique $R(t,\Theta_R)$ de la fonction de répartition complémentaire du temps de transit dans le tissu.

Au même titre que le modèle de fonction d'entrée artérielle, un tel modèle est stocké ou programmé dans l'unité de traitement 4 d'un système d'analyse tel que décrit en liaison avec les figures 1 ou 2.

A titre d'exemple, ce modèle de fonction de répartition complémentaire du temps de transit peut être un modèle dit « Gamma Intégral Corrigé » à deux paramètres :

$$
\begin{cases}
R\left(t,\Theta_R\right) = H\left(t\right) - \displaystyle\int_0^t h\left(\tau,MTT,\beta\right)d\tau \\[4mm]
h\left(\tau,MTT,\beta\right) = \dfrac{t^{\frac{MTT}{\beta}-1} e^{-t/\beta}}{\beta^{\frac{MTT}{\beta}}\Gamma\left(\dfrac{MTT}{\beta}\right)} \quad MTT>0, \beta>0 \\[4mm]
\Theta_R = \left(MTT,\beta\right) \\[2mm]
H(\ ) : \text{fonction généralisée créneau de Heaviside} \\[1mm]
\Gamma(\ ) : \text{fonction Gamma d'Euler}
\end{cases}
$$

En introduisant seulement des modèles paramétriques ou semi-paramétriques pour la fonction d'entrée artérielle et pour la fonction de répartition complémentaire des travaux tels que relatés dans le document « Estimation of Cerebral Blood Flow from Susceptibility Contrast MRI Using a Tissue Model - Shimony et al. » ont montré que l'on peut d'obtenir des résultats encourageants. Toutefois, ces travaux supposent des contraintes imposées notamment sur des paramètres du modèle de la fonction d'entrée artérielle improbables ou ineptes d'un point de vue physiologique diminuant ainsi la crédibilité des estimations obtenues. L'invention prévoit d'adapter une telle solution pour particulièrement améliorer la précision de l'estimation obtenue. En effet, un modèle standard de perfusion, par exemple en imagerie par Résonance Magnétique Nucléaire, peut s'exprimer de la manière équivalente suivante :

$$M : \begin{cases} S(t) = S_0 e^{-k \cdot TE \cdot C(t)} \\ C(t) = (\lambda.BF) \dfrac{C_a(t,\Theta_a)}{\lambda} \otimes R(t,\Theta_R) \end{cases}$$

$\lambda$ étant une constante non nulle.

Ainsi, si $\widehat{BF}$ et $\widehat{C_a(t,\Theta_a)}$ sont deux estimations permettant d'ajuster le modèle théorique de perfusion aux données expérimentales, alors $\lambda \widehat{BF}$ et $\dfrac{\widehat{C_a(t,\Theta_a)}}{\lambda}$ sont deux autres estimations ajustant tout aussi bien le modèle aux données expérimentales pour tout $\lambda \neq 0$.

Autrement dit, $BF$ et $C_a(t,\Theta_a)$ sont déterminés seulement à une constante multiplicative près sous un modèle standard de perfusion. La méthode de Bayes notamment permet toutefois d'obtenir des résultats satisfaisants car elle permet de tenir compte d'informations dont on dispose a priori sur les quantités manipulées qui rendent certaines valeurs desdites quantités, en particulier $BF$ et $C_a(t,\Theta_a)$, plus probables. Par conséquent certaines valeurs de la constante multiplicative $\lambda$ sont elles-mêmes plus probables ce qui a pour effet de rendre le problème de l'estimation des quantités mieux déterminé.

Cependant pour rendre ce problème parfaitement déterminé et ainsi améliorer la précision des estimations de paramètres tels que $BF$ ou $\Theta_a$, l'invention prévoit d'introduire une contrainte supplémentaire $\Psi(\Theta_a)$ sur le modèle de la fonction d'entrée artérielle $C_a(t,\Theta_a)$. Cette troisième relation peut exprimer par exemple la conservation d'une quantité physique sur toutes les fonctions d'entrée artérielle d'intérêt, telle que la masse totale d'agent de contraste circulant dans un voxel artériel au cours du temps. De manière particulièrement avantageuse, cette contrainte est indépendante du modèle $C_a(t,\Theta_a)$ considéré.

Cette contrainte peut s'exprimer, de manière générale, par une relation entre les paramètres $\Theta_a$ du modèle de la fonction d'entrée artérielle telle que $\Psi(\Theta_a)=0$.

Cette relation supplémentaire sous la forme d'une contrainte sur le modèle de la fonction d'entrée artérielle $C_a(t,\Theta_a)$ permet de fixer, une fois pour toute et pour tous les voxels considérés, la constante $\lambda$. Cette troisième relation est stockée ou programmée dans l'unité de traitement 4 d'un système d'analyse tel que décrit en liaison avec les figures 1 ou 2. L'ensemble des trois relations constituent un modèle global de perfusion contraint.

Le problème de l'estimation conjointe des paramètres $\Theta$ du modèle global contraint, en particulier de $BF$ et $\Theta_a$, devient alors tout-à-fait bien déterminé et posé.

Selon l'invention, cette contrainte $\Psi(\Theta_a)=0$ s'exprime comme :

$$\int_0^{+\infty} C_a(t,\Theta_a) dt = C_0$$

où $C_0$ est une constante arbitraire non nulle identique pour tous les voxels d'intérêt.

Dans le cas du modèle « tri-Gamma » pour une fonction d'entrée artérielle de paramètres $\Theta_a=(a,b,c,\alpha_0,\beta_0,t_0,\alpha_1,\beta_1,t_1,\alpha_2,\beta_2,t_2)$, cette contrainte sur les paramètres peut être exprimée comme $a+b+c=C_0$.

On a donc la relation :

$$\Psi(\Theta_a) = a + b + c - C_0 \ .$$

Sous cette contrainte, on peut ainsi éliminer l'un des trois paramètres, par exemple c en posant $c=C_0-a-b$, et se ramener à un modèle $C_a(t,\Theta_a)$ contraint à onze paramètres libres, tel que :

$$\begin{cases} C_a(t,\Theta_a) = \dfrac{a(t-t_0)^{\alpha_0-1} e^{-(t-t_0)/\beta_0}}{\beta_0^{\alpha_0}\Gamma(\alpha_0)} + \dfrac{b(t-t_1)^{\alpha_1-1} e^{-(t-t_1)/\beta_1}}{\beta_1^{\alpha_1}\Gamma(\alpha_1)} + \dfrac{(C_0-a-b)(t-t_2)^{\alpha_2-1} e^{-(t-t_2)/\beta_2}}{\beta_2^{\alpha_2}\Gamma(\alpha_2)} \\ \Theta_a = \left(a,b,\alpha_0,\beta_0,t_0,\alpha_1,\beta_1,t_1,\alpha_2,\beta_2,t_2\right) \\ \Gamma(\ ) \text{ fonction Gamma d'Euler} \end{cases}$$

L'invention permet ainsi de se ramener à un problème d'estimation conjointe des paramètres $\Theta=(\Theta_a,\Theta_R,BF,S_0,\Theta_B)$ en imagerie par résonance magnétique ou $\Theta=(\Theta_a,\Theta_R,BF,\Theta_B)$ en tomodensitométrie, où $\Theta_B$ est un vecteur de paramètres caractérisant les bruits de mesure sur le signal de perfusion S(t), sans avoir à fournir ou à estimer préalablement une quelconque fonction d'entrée d'artérielle.

L'invention se fonde ainsi sur un modèle global de perfusion éventuellement contraint comprenant :

- une première relation entre un signal de perfusion $S(t)$ et une concentration $C(t)$ d'un agent de contraste circulant dans un voxel de tissu au cours du temps t ;
- une deuxième relation entre ladite concentration $C(t)$, le flux sanguin $BF$, un modèle paramétrique ou semi-paramétrique $C_a(t,\Theta_a)$ d'une fonction d'entrée artérielle et un modèle paramétrique ou semi-paramétrique $R(t,\Theta_R)$ d'une fonction de répartition complémentaire du temps de transit dans le voxel, $\Theta_a$ et $\Theta_R$ étant respectivement les paramètres desdits modèles ;
- une troisième relation $\Psi(\Theta_a)=0$ entre les paramètres $\Theta_a$ du modèle de la fonction d'entrée artérielle $C_a(t,\Theta_a)$.

On pourra considérer les modèles respectivement d'une fonction d'entrée artérielle $C_a(t,\Theta_a)$ et d'une fonction de répartition complémentaire du temps de transit $R(t,\Theta_R)$ comme des sous-modèles d'un modèle global de perfusion contraint par ladite troisième relation $\Psi(\Theta_a)=0$ entre les paramètres $\Theta_a$ dudit modèle de la fonction d'entrée artérielle $C_a(t,\Theta_a)$.

A titre d'exemple en imagerie par Résonance Magnétique Nucléaire, un tel modèle global de perfusion contraint $M$ peut être exprimé sous une forme telle que :

$$M : \begin{cases} S(t) = S_0 e^{-k\cdot TE\cdot C(t)} \\ C(t) = \eta.BF \cdot C_a(t,\Theta_a) \otimes R(t,\Theta_R) \\ \Psi(\Theta_a) = 0 \\ \Theta = \left(\Theta_a,\Theta_R,BF,S_0,\Theta_B\right) \end{cases}$$

où $\otimes$ désigne le produit de convolution, $\eta$ est une constante non nulle fixée arbitrairement et $BF$ est le flux sanguin circulant dans le voxel considéré.

De même, en imagerie par tomodensitométrie un modèle global de perfusion contraint $M$ peut être exprimé sous une forme telle que :

$$M : \begin{cases} S(t) = \alpha.C(t) \\ C(t) = \eta.BF \cdot C_a(t,\Theta_a) \otimes R(t,\Theta_R) \\ \Psi(\Theta_a) = 0 \\ \Theta = \left(\Theta_a,\Theta_R,BF,\Theta_B\right) \end{cases}$$

Où $\otimes$ désigne le produit de convolution, $\eta$ est une constante non nulle fixée arbitrairement, $BF$ est le flux sanguin circulant dans le voxel considéré et $\alpha$ une constante non nulle.

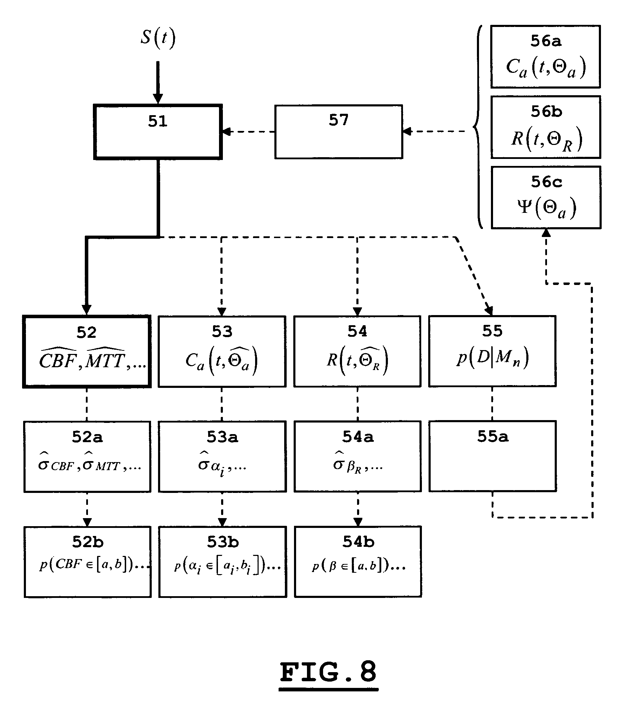

En liaison avec la figure 8, un procédé pour estimer des paramètres hémodynamiques, conforme à l'invention, comporte une étape 51 pour estimer conjointement les paramètres $\Theta$ d'un modèle $M$ à partir de signaux de perfusion $S(t)$. Un tel procédé est mis en oeuvre par une unité de traitement 4, telle que décrite en figures 1 ou 2, adaptée pour pouvoir notamment mettre en oeuvre des techniques mathématiques et numériques pour résoudre un problème d'estimation conjointe de paramètres.

A titre d'exemples, une telle unité de traitement 4 est apte à mettre en oeuvre les Méthodes du Maximum de Vraisemblance, les Méthodes des Moindres Carrés Nonlinéaires ou la Méthode de Bayes.

Afin d'illustrer un mode de réalisation préféré de l'invention, nous allons considérer que :

- l'unité de traitement 4 met en oeuvre la méthode de Bayes ;
- le modèle de la Fonction d'Entrée Artérielle, stocké ou programmé dans l'unité de traitement 4, est le modèle « tri-Gamma » ;
- le modèle la Fonction de Répartition Complémentaire, stocké ou programmé dans l'unité de traitement 4, est le modèle « Gamma Intégral Corrigé » ;
- la contrainte, stockée ou programmée dans l'unité de traitement 4, est $\Psi(\Theta_a)=C_0\text{-}a\text{-}b\text{-}c=0$.

Pour une application à l'imagerie de perfusion par Résonance Magnétique Nucléaire, le modèle $C_a(t,\Theta_a)$ contraint s'écrit donc :

$$\begin{cases} C_a\left(t,\Theta_a\right) = \dfrac{a\left(t-t_0\right)^{\alpha_0-1} e^{-\left(t-t_0\right)/\beta_0}}{\beta_0^{\alpha_0}\Gamma\left(\alpha_0\right)} + \dfrac{b\left(t-t_1\right)^{\alpha_1-1} e^{-\left(t-t_1\right)/\beta_1}}{\beta_1^{\alpha_1}\Gamma\left(\alpha_1\right)} + \dfrac{\left(C_0-a-b\right)\left(t-t_2\right)^{\alpha_2-1} e^{-\left(t-t_2\right)/\beta_2}}{\beta_2^{\alpha_2}\Gamma\left(\alpha_2\right)} \\[4mm] \Theta_a = \left(a,b,\alpha_0,\beta_0,t_0,\alpha_1,\beta_1,t_1,\alpha_2,\beta_2,t_2\right) \\[2mm] \Gamma\left(\ \right) \text{ fonction Gamma d'Euler} \end{cases}$$

Soit $t_i, i=1, N$ des instants d'échantillonnage et $S(t_i), i=1, N$ l'intensité du signal de perfusion mesurée en ces instants. Les données expérimentales sont donc $D=[S(t_1),...,S(t_N)]$.

Si l'on suppose un bruit de mesure additif, blanc, Gaussien, stationnaire et d'écart-type $\sigma=\Theta_B$, alors la fonction de vraisemblance ou distribution directe des données de perfusion $D$ sachant tous les paramètres s'écrit :

$$p\left(D|\Theta,M\right) = \left(2\pi\right)^{-\frac{N}{2}} \sigma^{-N} e^{-\frac{\sum\limits_{i=1}^{N}\left[S\left(t_i\right)-S_0 e^{-k\cdot TE\cdot C\left[t_i\right]}\right]^2}{2\sigma^2}}$$

$C(t)=\eta.BF\cdot C_a(t,\Theta_a)\otimes R(t,\Theta_R)$ est calculée numériquement après discrétisation temporelle soit, de préférence, analytiquement en utilisant éventuellement une approximation pour le produit de convolution de deux distributions de probabilité $\Gamma$.

Etant donnée une distribution conjointe a priori de tous les paramètres sachant le modèle $p(\Theta|M)$ représentant l'information dont on dispose avant l'expérience sur ces paramètres, on obtient une distribution conjointe a posteriori par la règle de Bayes :

$$p\left(\Theta|D,M\right) = \frac{p\left(\Theta|M\right)p\left(D|\Theta,M\right)}{\int\limits_{\Theta} p\left(\Theta|M\right)p\left(D|\Theta,M\right)d\Theta} \propto p\left(\Theta|M\right)p\left(D|\Theta,M\right)$$

A partir de cette distribution conjointe, le procédé selon l'invention permet d'obtenir 52 la distribution marginale a posteriori de chacun des paramètres hémodynamiques $\theta$ d'intérêt tel que le flux sanguin $BF$, le volume sanguin $BV$ ou le temps de transit moyen $MTT$, en marginalisant tous les autres. A partir de cette distribution marginale, on peut enfin obtenir un estimateur du paramètre, par exemple l'estimateur de Bayes sous fonction de coût quadratique en prenant l'espérance mathématique de cette distribution ou encore la valeur la plus probable du paramètre (estimateur du maximum a posteriori). Ainsi l'invention permet par exemple de pouvoir estimer pour tout voxel d'intérêt, une estimation du flux sanguin $\widehat{CBF}$ d'un cerveau, une estimation du volume sanguin $\widehat{CBV}$ ainsi qu'une estimation du Temps de Transit Moyen $\widehat{MTT}$.

De manière optionnelle, un procédé conforme à l'invention permet d'obtenir 52a des informations complémentaires sous la forme d'intervalles de confiance sur des paramètres estimés et même des paris 52b sur lesdits intervalles de confiance. Ces informations complémentaires peuvent être particulièrement utiles, pour un utilisateur tel qu'un praticien, et se révéler être une aide supplémentaire dans l'élaboration d'un diagnostic. A l'aide des techniques de l'art antérieur à l'invention, un praticien n'a aucune idée de la précision des estimations dont il dispose pour chaque voxel d'intérêt.

Par exemple, pour un paramètre hémodynamique particulier $\theta$ - tel que le flux cérébral sanguin $\theta = CBF$ par exemple, l'invention permet d'obtenir une distribution marginale a posteriori :

$$p\left(\theta\middle|D,M\right) = \int_{\Theta\backslash\theta} p\left(\Theta\middle|D,M\right) d\left(\Theta\backslash\theta\right)$$

puis un Estimateur de Bayes $\hat{\theta}$ de $\theta$ sous fonction de coût quadratique $L(\theta\text{-}\hat{\theta}^Q)=(\theta\text{-}\hat{\theta}^Q)^2$ :

$$\hat{\theta}^Q = \int_\theta \theta.p\left(\theta\middle|D,M\right)d\theta$$

ou la valeur la plus probable du paramètre :

$$\hat{\theta}^P = \arg\max_\theta p\left(\theta\middle|D,M\right)$$

L'unité 4 peut aussi déduire optionnellement :

- la variance de l'estimateur de Bayes sous fonction de coût quadratique :

$$\widehat{\sigma}_\theta^2 = \int_\theta \left(\theta - \hat{\theta}^Q\right)^2.p\left(\theta\middle|D,M\right)d\theta$$

- un intervalle de confiance, par exemple l'intervalle de confiance à $\pm 1\sigma$ :

$$\left[\hat{\theta} - \widehat{\sigma}_\theta, \hat{\theta} + \widehat{\sigma}_\theta\right]$$

Selon un mode de réalisation, l'unité de traitement 4 mettant en oeuvre un procédé selon l'invention, permet de calculer 52b une probabilité/taux de pari que le paramètre soit dans l'intervalle de confiance, par exemple :

$$p\left(\theta \in \left[\hat{\theta} - \widehat{\sigma}_\theta, \hat{\theta} + \widehat{\sigma}_\theta\right]\right) = \int_{\hat{\theta}-\widehat{\sigma}_\theta}^{\hat{\theta}+\widehat{\sigma}_\theta} p\left(\theta\middle|D,M\right)d\theta$$

En variante, l'invention prévoit que le procédé permette d'obtenir 53 une information complémentaire correspondant à la distribution marginale conjointe a posteriori des paramètres de la fonction d'entrée artérielle locale pour un volume de tissu considéré, telle que :

$$p\left(\Theta_a\big|D,M\right)=\int_{\Theta\backslash\Theta_a} p\left(\Theta\big|D,M\right)d\left(\Theta\backslash\Theta_a\right)$$

L'invention permet ainsi obtenir par exemple une fonction d'entrée artérielle « moyenne » $C_a\left(t,\widehat{\Theta_a}\right)$ sous

fonction de coût quadratique $L\left(\Theta_a-\widehat{\Theta_a}^Q\right)=\left\|\Theta_a-\widehat{\Theta_a}^Q\right\|^2$ , de paramètres conjoints :

$$\widehat{\Theta_a}^Q=\int_{\Theta_a}\Theta_a\cdot p\left(\Theta_a\big|D,M\right)d\Theta_a$$

ou encore une fonction d'entrée artérielle « la plus probable » de paramètres :

$$\widehat{\Theta_a}^P=\arg\max_{\Theta_a} p\left(\Theta_a\big|D,M\right)$$

De la même manière, l'invention prévoit une variante de réalisation pour laquelle un procédé conforme à l'invention comporte une étape 54 pour calculer une fonction de répartition complémentaire « moyenne » ou « la plus probable » $R\left(t,\widehat{\Theta_R}\right)$ .

On peut ainsi en outre, obtenir également une information complémentaire sous la forme d'estimations de la concentration de l'agent de contraste par $C\left(t,\widehat{\Theta}\right)$ .

Selon un mode de réalisation, l'invention prévoit que ces fonctions d'entrée artérielle ou fonctions de répartition complémentaire estimées, « moyennes » ou « plus probables », puissent être comparées à celle(s) obtenue(s) par les approches antérieures à l'invention ou encore à celles obtenues à partir de modèles de fonction d'entrée artérielle ou de fonction de répartition complémentaire distincts dans le but de permettre la sélection des modèles les plus pertinents.
En effet, il peut être intéressant de comparer les fonctions d'entrée artérielle estimées à la véritable fonction d'entrée artérielle lorsque celle-ci est connue et tâcher de minimiser leur écart en introduisant des modèles plus appropriés, si besoin est.
En liaison avec la figure 8, l'invention prévoit en outre la possibilité qu'un procédé conforme à l'invention puisse comporter une ou plusieurs étapes complémentaires pour obtenir 53a des intervalles de confiance voire pour obtenir 53b des paris sur ces intervalles pour les paramètres des fonctions d'entrée artérielle. Il peut en être de même, pour obtenir 54a des intervalles de confiance voire pour obtenir 54b des paris sur ces intervalles pour les paramètres des fonctions répartition complémentaire.
Les dimensions des modèles paramétriques et semi-paramétriques typiques pour les fonctions d'entrée artérielle $C_a(t,\Theta_a)$ et les fonctions de répartition complémentaire $R(t,\Theta_R)$ sont suffisamment petites pour que puissent s'appliquer des méthodes d'échantillonnage ou d'approximation de la distribution conjointe a posteriori $p(\Theta|D,M)$ et des distributions marginales a posteriori telles que les méthodes de Monte-Carlo à Chaînes de Markov ou les Méthodes de Bayes Variationnelles.
L'invention permet d'obtenir dès lors des approximations des estimations souhaitées pour les paramètres d'intérêt.
Selon un mode de réalisation particulier, l'invention prévoit que le procédé, décrit en liaison avec la figure 8 et mis en oeuvre par une unité de traitement 4 adaptée en conséquence, puisse quantifier l'adéquation du modèle global de perfusion aux données expérimentales D par le calcul 55 de la probabilité de ces données sachant le modèle

$$p\left(D\big|M\right)=\int_{\Theta} p\left(\Theta\big|M\right)p\left(D\big|\Theta,M\right)d\Theta,$$ avec $\Theta=\{\Theta_a,\Theta_R,\theta,S_0,\Theta_B\}$ en imagerie par résonance magnétique ou

$\Theta=(\Theta_a,\Theta_R,BF,\Theta_B)$ en tomodensitométrie.
Le calcul 55 de cette quantité n'était pas concevable avec les techniques de l'art antérieur à l'invention puisque selon ces dernières, on ne dispose pas de modèles globaux de signaux bien déterminés.

L'invention prévoit que cette quantité puisse permettre de comparer de manière objective plusieurs modèles de perfusion globaux contraints obtenus à partir de différents sous-modèles pour la fonction d'entrée d'artérielle $C_a(t,\Theta_a)$ et/ou de différents sous-modèles pour la fonction de répartition complémentaire $R(t,\Theta_R)$ et de différentes contraintes supplémentaires $\Psi(\Theta_a)=0$ tels que décrits précédemment.

Ainsi, l'invention prévoit que le procédé puisse être mis en oeuvre par l'unité de traitement par itérations successives en utilisant des modèles globaux de perfusion contraints distincts. L'unité de traitement peut conserver le résultat du calcul 55 pour chaque modèle utilisé. L'invention permet de sélectionner au final le meilleur modèle global de perfusion. Ce modèle est celui pour lequel la probabilité des signaux de perfusion sachant le modèle est la plus grande en moyenne sur l'ensemble des voxels d'intérêt.

En variante, l'invention prévoit que le procédé puisse comporter des étapes 56a, 56b et 56c pour choisir lors d'une itération respectivement un sous-modèle $C_a(t,\Theta_a)$, un sous-modèle $R(t,\Theta_R)$, et une contrainte $\Psi(\Theta_a)=0$ parmi une pluralité et ainsi ajuster 57 un modèle global de perfusion. Cette sélection peut être réalisée manuellement par un utilisateur 6 au moyen de paramètres 16 ou automatiquement par le procédé lui-même. Par un processus itératif et essais successifs, il devient possible grâce à l'invention de progresser dans la modélisation et la compréhension des phénomènes de perfusion et à terme d'identifier et de valider les meilleurs modèles en fonction des patients, des pathologies, des types de tissus, etc.

A titre d'exemple d'application de l'invention, nous pouvons citer les principales étapes de mise en oeuvre de l'invention au moyen d'un système d'analyse d'imagerie de perfusion adapté tel que celui décrit en figures 1 ou 2 :

- ouverture d'un dossier patient ou prise en compte de séquences d'images par l'unité de traitement 4 (ou de prétraitement 7) pour sélectionner des séquences d'images d'intérêt - en particulier, sélection des images I1 à In de perfusion au cours du temps à partir desquelles sont obtenus les signaux de perfusion $S(t)$ pour chaque voxel, tel qu'illustré en figure 5a ;

- prévisualisation au moyen d'une interface homme-machine 5 des images pour permettre à un utilisateur 6 d'identifier des tranches ou des zones d'intérêt ;

- estimation conjointe par l'unité de traitement 4 des paramètres hémodynamiques 14, tels que $\widehat{CBF}$ ou $\widehat{MTT}$ , pour un organe tel que le cerveau humain et délivrance desdits paramètres 14 à l'interface homme-machine 5 pour que celle-ci les présente in fine sous la forme de cartes où l'intensité ou la couleur de chaque pixel dépend de la valeur calculée, par exemple de manière linéaire ; cette étape peut inclure également la délivrance d'autres quantités estimées tels que des intervalles de confiance, des paris, ou autres informations complémentaires associées aux paramètres du modèle de perfusion global;

- affichage desdites cartes pour en restituer la teneur à l'utilisateur 6 ;

- seuillage et filtrage sous l'impulsion 16 de l'utilisateur 6 pour ne conserver qu'une région digne d'intérêt et/ou supprimer certaines aberrations ;

- sélection assistée de ladite zone pathologique d'intérêt par l'utilisateur, caractérisée par une anomalie de la distribution d'un ou de plusieurs paramètres hémodynamiques 14 ;

- estimation, par l'unité de traitement 4, du volume de la zone de tissus anormalement perfusée et éventuellement de certaines quantités telles que le ratio des volumes des zones lésées et anormalement perfusées, sur lesquelles un praticien pourra peaufiner son diagnostic et sa prise de décision thérapeutique (thrombolyse intraveineuse afin de résorber un caillot sanguin par exemple).

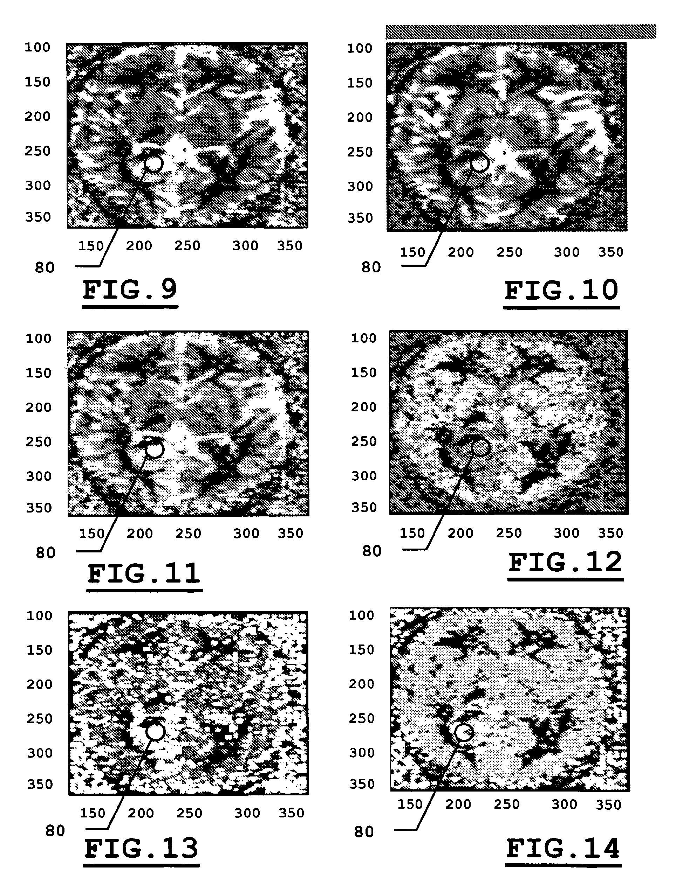

Les figures 9 à 17 permettent d'illustrer un mode d'affichage sous la forme de cartes, des paramètres hémodynamiques 14 estimés conformément à l'invention voire des écarts-types ou probabilité qui leur sont associés.

Ainsi pour un cerveau humain analysé à l'aide d'imagerie par Résonance Magnétique Nucléaire, la figure 9 permet de visualiser une estimation des volumes sanguins cérébraux *CBV*. Une telle carte permet de mettre en évidence une zone ischémique probable 80. En effet, il est possible de constater à l'aide d'une interface 6 adaptée, une nette augmentation du paramètre *CBV* dans le territoire de l'artère cérébrale postérieure droite par rapport à l'hémisphère contralatérale. Une vasodilatation consécutive à l'ischémie peut être révélée par une lecture de la carte telle qu'illustrée en figure 9.

La figure 10 permet d'illustrer une carte relative à l'estimation des flux sanguins cérébraux en cas d'ischémie cérébrale. On peut constater en analysant la carte, une légère diminution du paramètre *CBF* dans le territoire de l'artère cérébrale postérieure droite par rapport à l'hémisphère contralatérale consécutive à l'ischémie.

La figure 11 permet d'illustrer une carte relative à l'estimation des temps de transit moyen *MTT*. On peut constater en analysant la carte, une nette augmentation des *MTT* dans le territoire 80 de l'artère cérébrale postérieure droite par rapport à l'hémisphère contralatérale consécutive à l'ischémie.

La figure 12 permet de décrire une carte relative à l'estimation du paramètre *a* d'un modèle de fonction d'entrée artérielle « tri-Gamma » à 12 paramètres conforme à l'invention. On peut constater une nette diminution de *a* dans le territoire 80 de l'artère cérébrale postérieure droite par rapport à l'hémisphère contralatérale. Cette information permet de constater une diminution relative de la quantité d'agent de contraste lors de la circulation par rapport à la quantité pendant la recirculation. Cette information n'est pas disponible avec les techniques selon l'art antérieur à l'invention.

La figure 13 permet de décrire une carte relative à l'estimation du paramètre $\beta_2$ du modèle de fonction d'entrée artérielle « tri-Gamma » à 12 paramètres conforme à l'invention. On peut constater en analysant la carte, une nette augmentation de $\beta_2$ dans le territoire 80 de l'artère cérébrale postérieure droite par rapport à l'hémisphère contralatérale. Cette information n'est pas disponible avec les techniques selon l'art antérieur à l'invention.

La figure 14 permet de décrire une carte relative à l'estimation du paramètre $t_1$ du modèle de fonction d'entrée artérielle « tri-Gamma » à 12 paramètres conforme à l'invention. On peut constater en analysant la carte, une nette augmentation de $t_1$ dans le territoire de l'artère cérébrale postérieure droite par rapport à l'hémisphère contralatérale. Cette information n'est pas disponible avec les techniques selon l'art antérieur à l'invention.

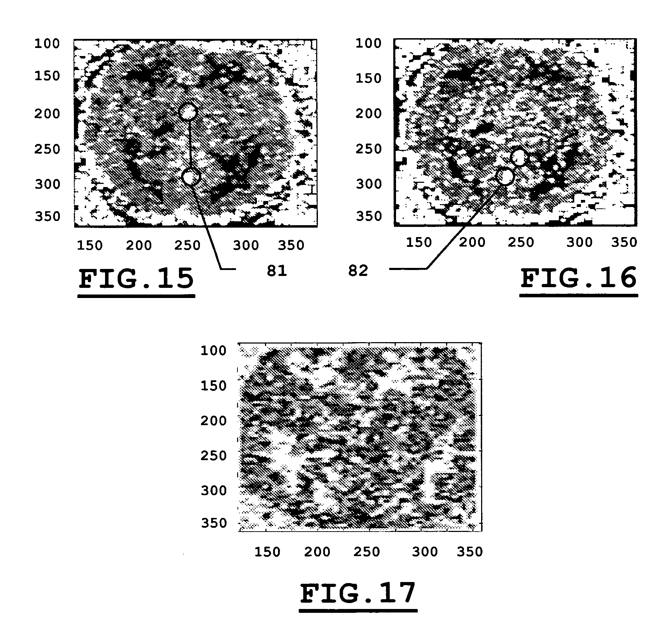

La figure 15 permet de décrire une carte relative à l'estimation de l'écart-type des flux sanguins cérébraux estimés $\sigma_{CBF}$ à partir du modèle de fonction d'entrée artérielle « tri-Gamma » à douze paramètres conforme à l'invention. On peut constater en analysant la carte, que les écarts-types sont les plus élevés dans des zones 81 ne contenant pas de tissu mais des artères. Les écarts-types ne sont pas plus élevés dans la zone ischémique que dans la zone contralatérale, confortant l'estimation des *CBF* dans cette zone, telle que décrite par la figure 10. Cette information n'est pas disponible avec les techniques selon l'art antérieur à l'invention.

La figure 16 permet de décrire une carte relative à l'estimation de l'écart-type des temps de transit moyen estimés $\sigma_{MTT}$ à partir du modèle de fonction de répartition complémentaire « intégral Gamma corrigé » à deux paramètres conforme à l'invention. On peut constater en analysant la carte, que les écarts-types sont les plus élevés dans des zones 82 ne contenant pas de tissu mais des artères. Il s'agit précisément de zones similaires à celles où les écarts-types des flux cérébraux sanguins sont également les plus élevés. Les écarts-types ne sont pas plus élevés dans la zone ischémique que dans la zone contralatérale, confortant l'estimation des *MTT* dans cette zone, estimation telle que décrite par la figure 11. Cette information n'est pas disponible avec les techniques selon l'art antérieur à l'invention.

La figure 17 permet de décrire une carte relative à l'estimation de la probabilité que le flux sanguin cérébral soit dans l'intervalle de confiance $\left[ \widehat{CBF} - \hat{\sigma}_{CBF}, \widehat{CBF} + \hat{\sigma}_{CBF} \right]$ sous le modèle de fonction d'entrée artérielle « tri-Gamma » à douze paramètres conforme à l'invention. On peut constater en analysant la carte que, mis à part des ajustements aberrants, les probabilités sont centrées autour de 0,68. C'est une valeur à laquelle on est en droit de s'attendre si la distribution de probabilité a posteriori de *CBF* suit une loi normale.

Grâce aux cartes présentées précédemment, l'invention permet de mettre à la disposition d'un utilisateur tout un ensemble d'informations utiles, informations qui ne pouvaient être disponibles à l'aide des techniques connues de l'état de l'art. Cette mise à disposition est rendue possible par une adaptation de l'unité de traitement 4 selon les figures 1 ou 2 en ce que ses moyens pour communiquer avec le monde extérieur de l'unité 4 sont aptes à délivrer les paramètres estimés 14 selon un format approprié à une interface homme-machine 5 apte à restituer à un utilisateur 6 lesdits paramètres estimés sous la forme par exemple de cartes telles qu'illustrées par les figures 9 à 17.

Nous pouvons remarquer en outre que l'invention permet de supprimer des étapes nécessaires selon les techniques de l'art antérieur à l'invention. Parmi diverses étapes nous pouvons citer la suppression de :

- la sélection manuelle ou automatique ou détermination d'une fonction d'entrée artérielle globale ou de fonctions d'entrée artérielle locales ;
- la mise en oeuvre par l'unité de traitement d'un algorithme de déconvolution du modèle standard de perfusion $C(t) = \eta \cdot BF \cdot C_a(t) \otimes R(t)$ pour chaque voxel, sur la base de ou desdites fonction(s) d'entrée artérielle(s) sélectionnée(s), afin d'obtenir une estimation de $BF.R(t)$.

Nous pouvons remarquer en outre que selon l'invention, l'unité de traitement est adaptée pour mettre en oeuvre un algorithme d'estimation conjointe des paramètres du modèle de perfusion global en lieu et place d'un algorithme de déconvolution conformément aux techniques antérieures.

Grâce à l'invention, les informations délivrées sont plus nombreuses et justes. Les informations dont dispose un chercheur ou un praticien sont ainsi de nature à accroître la confiance de ce dernier dans la détermination d'un diagnostic et d'une décision thérapeutique.

Pour améliorer les performances du système selon l'invention, celle-ci prévoit que l'unité de traitement 4 puisse être dotée de moyens pour paralléliser des calculs sur les voxels de l'image pour lesquels l'estimation des paramètres est requise. Une telle unité de traitement peut comporter des moyens tels que des microprocesseurs graphiques *(Graphical Processor Unit* (GPU) en langue anglaise) ou mettre en oeuvre des grappes de calcul *(clusters,* en langue anglaise). En variante une telle unité de traitement peut mettre en oeuvre des moyens programmés tels que les Méthodes de Monte-Carlo parallèles. En variante, l'unité de traitement conforme à l'invention peut s'appuyer sur des moyens de calcul distants. Les temps de calculs peuvent ainsi être considérablement réduits. En outre, une unité de traitement conforme à l'invention peut comporter des moyens pour mémoriser un ou plusieurs modèle(s) global(aux) de perfusion, ou une pluralité de sous-modèles de fonction d'entrée artérielle ou de fonction de répartition complémentaire du temps de transit et une pluralité de contraintes basées sur les paramètres desdits sous-modèles de fonction d'entrée artérielle. En variante une telle unité de traitement peut autoriser un chargement de tels modèles globaux de perfusion contraints ou de sous-modèles et de contraintes par l'intermédiaire de ses moyens pour communiquer avec le monde extérieur. Ces derniers peuvent être aptes à mettre en oeuvre une communication filaire ou distante selon des techniques connues de l'état de l'art.

L'invention a été décrite et illustrée dans le domaine de l'Imagerie de Perfusion par Susceptibilité de Contraste Dynamique *(Dynamic Susceptibility Contrast Magnetic Resonance Imaging* (DSC-MRI) en langue anglaise). L'invention ne saurait être limitée à ce seul exemple d'application mais peut s'appliquer également à d'autres technologies médicales telles que l'imagerie de perfusion par Tomodensitométrie notamment.

**Revendications**

1.  Procédé pour estimer un ou plusieurs paramètres hémodynamiques de perfusion (14) d'un volume élémentaire

    - dit voxel - d'un organe, ledit procédé étant mis en oeuvre par une unité de traitement (4) d'un système d'analyse d'imagerie de perfusion, et comportant une étape pour estimer un ou plusieurs paramètres hémodynamiques à partir de signaux (15) de perfusion $S(t)$, l'étape pour estimer le ou lesdits paramètres hémodynamiques consistant en l'évaluation d'une distribution marginale a posteriori (52) pour le ou lesdits paramètres hémodynamiques par l'assignation (51) préalable d'une distribution conjointe a priori des paramètres $\Theta$ d'un modèle global de perfusion comportant :

      - une première relation entre le signal de perfusion $S(t)$ et une concentration $C(t)$ d'un agent de contraste circulant dans ledit voxel au cours du temps t *;*
      - une deuxième relation entre la concentration C(t) d'un agent de contraste, le flux sanguin *BF,* un modèle paramétrique ou semi-paramétrique $C_a(t,\Theta_a)$ d'une fonction d'entrée artérielle et un modèle paramétrique ou semi-paramétrique $R(t,\Theta_R)$ d'une fonction de répartition complémentaire du temps de transit dans le voxel, $\Theta_a$ et $\Theta_R$ étant respectivement les paramètres desdits modèles $C_a(t,\Theta_a)$ et $R(t,\Theta_R)$ ;

      **caractérisé en ce que** ledit modèle global de perfusion comporte en outre une troisième relation $\Psi(\Theta_a)=0$ entre les paramètres $\Theta_a$ du modèle de la fonction d'entrée artérielle $C_a(t,\Theta_a)$ consistant à exprimer l'un desdits paramètres en fonction des autres, ladite troisième relation étant identique pour tout voxel d'intérêt et s'exprimant com-

      me: $\int_0^{+\infty} C_a(t,\Theta_a)\,dt = C_0$  où $C_0$ est une constante arbitraire non 0 nulle, identique pour tout voxel d'intérêt.

2.  Procédé selon la revendication 1 **caractérisé en ce que** :

    - le signal de perfusion $S(t)$ a été préalablement obtenu partir d'images numériques (12, 13) délivrées par un appareil (1) d'imagerie de perfusion par résonance magnétique ;
    - la première relation du modèle global de perfusion s'exprime par $S(t)=S_0 e^{-k \cdot TE \cdot C(t)}$ où $S_0$ est l'intensité moyenne du signal avant l'arrivée de l'agent de contraste dans le voxel, *TE* est un temps d'écho et *k* une constante non nulle ;
    - la deuxième relation du modèle global de perfusion s'exprime par $C(t)=\eta.BF \cdot C_a(t,\Theta_a) \otimes R(t,\Theta_R)$ où $\otimes$ désigne le produit de convolutionet $\eta$ est une constante non nulle.

3.  Procédé selon la revendication 1 **caractérisé en ce que** :

    - le signal de perfusion $S(t)$ a été préalablement obtenu à partir d'images numériques (12, 13) délivrées par un appareil (1) d'imagerie de perfusion par tomodensitométrie ;
    - la première relation du modèle global de perfusion est une relation de proportionnalité $S(t)=\alpha.C(t)$ où $\alpha$ est une constante non nulle;

- la deuxième relation du modèle global de perfusion s'exprime par $C(t)=\eta.BF\cdot C_a(t,\Theta_a)\otimes R(t,\Theta_R)$ où $\otimes$ désigne le produit de convolutionet $\eta$ est une constante non nulle.

4. Procédé selon l'une quelconque des revendications précédentes **caractérisé en ce qu'**il est mis en oeuvre par itérations successives pour une pluralité de voxels considérés.

5. Procédé selon l'une des revendications précédentes **caractérisé en ce que** l'estimation (51) conjointe des paramètres $\Theta$ du modèle global de perfusion mise en oeuvre par l'unité de traitement (4) est réalisée au moyen de la méthode de Bayes ou des méthodes du Maximum de Vraisemblance ou encore des méthodes des Moindres Carrés nonlinéaires.

6. Procédé selon l'une des revendications précédentes **caractérisé en ce qu'**il comporte une étape (55) pour quantifier l'adéquation du modèle global de perfusion àdes données de perfusion $D=[S(t_1),...,S(t_N)]$ expérimentalespar un calcul d'une probabilité $p\big(D\big|M\big)=\int_{\Theta} p\big(\Theta\big|M\big)p\big(D\big|\Theta,M\big)d\Theta$ de cesdonnées sachant ledit modèle.

7. Procédé selon l'une quelconque des revendications précédentes **caractérisé en ce qu'**il comporte une étape préalable (56a, 56b, 56c, 57) pour choisir un modèle global de perfusion parmi une pluralité.

8. Procédé selon la revendication précédente **caractérisé en ce qu'**il est mis en oeuvre itérativement par l'unité de traitement (4) pour chaque modèle global de perfusion connu de ladite unité de traitement.

9. Procédé selon la revendication précédente lorsqu'elle dépend de la revendication 6 **caractérisé en ce que** sont (55a) délivrés les paramètres estimés selon le modèle de perfusion dont la probabilité sachant ledit modèle est la plus grande.

10. Procédé selon l'une des revendications précédentes **caractérisé en ce que** le modèle $C_a(t,\Theta_a)$ d'une fonction d'entrée artérielle est un modèle dit « tri-Gamma » à douze paramètres défini par :

$$\begin{cases} C_a\big(t,\Theta_a\big) = \dfrac{a\big(t-t_0\big)^{\alpha_0-1}e^{-\big(t-t_0\big)/\beta_0}}{\beta_0^{\alpha_0}\Gamma\big(\alpha_0\big)} + \dfrac{b\big(t-t_1\big)^{\alpha_1-1}e^{-\big(t-t_1\big)/\beta_1}}{\beta_1^{\alpha_1}\Gamma\big(\alpha_1\big)} + \dfrac{c\big(t-t_2\big)^{\alpha_2-1}e^{-\big(t-t_2\big)/\beta_2}}{\beta_2^{\alpha_2}\Gamma\big(\alpha_2\big)} \\ \Theta_a = \big(a,b,c,\alpha_0,\beta_0,t_0,\alpha_1,\beta_1,t_1,\alpha_2,\beta_2,t_2\big) \\ \Gamma\big(\ \big) : \text{fonction Gamma d'Euler} \end{cases}$$

11. Procédé selon l'une des revendications précédentes **caractérisé en ce que** le modèle $R(t,\Theta_R)$ d'une fonction de répartition complémentaire du temps de transit dans le voxel est un modèle dit « Gamma Intégral Corrigé » à deux paramètres défini par :

$$\begin{cases} R\left(t,\Theta_R\right) = H\left(t\right) - \int_0^t h\left(\tau,MTT,\beta\right)d\tau \\[2em] h\left(\tau,MTT,\beta\right) = \dfrac{\tau^{\frac{MTT}{\beta}-1} e^{-\tau/\beta}}{\beta^{\frac{MTT}{\beta}} \Gamma\left(\dfrac{MTT}{\beta}\right)} \quad MTT > 0, \beta > 0 \\[2em] \Theta_R = \left(MTT,\beta\right) \\[1em] H\left(\ \right) : \text{fonction généralisée créneau de Heaviside} \\[0.5em] \Gamma\left(\ \right) : \text{fonction Gamma d'Euler} \end{cases}$$

**12.** Procédé selon l'une quelconque des revendications précédentes **caractérisé en ce qu'**il comporte une étape (53) pour calculer une information complémentaire sous la forme d'une fonction d'entrée artérielle estimée $C_a\left(t,\widehat{\Theta_a}\right)$.

**13.** Procédé selon l'une quelconque des revendications précédentes **caractérisé en ce qu'**il comporte une étape (54) pour calculer une information complémentaire sous la forme d'une fonction de répartition complémentaire estimée $R\left(t,\widehat{\Theta_R}\right)$.

**14.** Procédé selon l'une quelconque des revendications précédentes **caractérisé en ce qu'**il comporte une étape (52a, 53a, 54a) pour calculer une information complémentaire sous la forme d'un intervalle de confiance associé à un paramètre du modèle global de perfusion.

**15.** Procédé selon l'une quelconque des revendications précédentes **caractérisé en ce qu'**il comporte une étape (52b, 53b, 53c) pour calculer une information complémentaire sous la forme d'un taux de pari associé à un paramètre du modèle global de perfusion.

**16.** Procédé selon l'une quelconque des revendications précédentes **caractérisé en ce qu'**il comporte une étape pour délivrer (52) un ou plusieurs paramètres hémodynamiques estimés (14) à une interface homme-machine (5) apte à restituer à un utilisateur (6) lesdits paramètres estimés.

**17.** Procédé selon l'une quelconque des revendications 13 à 16 **caractérisé en ce qu'**il comporte une étape pour délivrer (52) toute information complémentaire à une interface homme-machine (5) apte à restituer à un utilisateur (6) les paramètres estimés ainsi que ladite information complémentaire.

**18.** Unité de traitement (4) comportant des moyens de mémorisation, des moyens pour communiquer avec le monde extérieur et des moyens de traitement, **caractérisée en ce que** :

- les moyens pour communiquer sont aptes à recevoir du monde extérieur un signal $S(t)$ (15) obtenu par imagerie de perfusion ;
- les moyens de mémorisation comportent un modèle global de perfusion, ledit modèle comportant :

 - une première relation entre le signal de perfusion $S(t)$ et une concentration $C(t)$ d'un agent de contraste circulant dans ledit voxel au cours du temps t *;*
 - une deuxième relation entre le flux sanguin $BF$ dans le voxel, la concentration $C(t)$, un modèle paramétrique ou semi-paramétrique $C_a(t,\Theta_a)$ d'une fonction d'entrée artérielle et un modèle paramétrique ou semi-paramétrique $R(t,\Theta_R)$ d'une fonction de répartition complémentaire du temps de transit dans le voxel, $\Theta_a$ et $\Theta_R$ étant respectivement les paramètres desdits modèles ;
 - une troisième relation $\Psi(\Theta_a)=0$ entre les paramètres $\Theta_a$ du modèle de la fonction d'entrée artérielle $C_a(t,\Theta_a)$

consistant à exprimer l'un desdits paramètres en fonction des autres, ladite troisième relation étant identique pour tout voxel d'intérêt et s'exprimant comme: $\int_{0}^{+\infty} C_a(t,\Theta_a)\,dt = C_0$ où $C_0$ est une constante

arbitraire non 0 nulle, identique pour tout voxel d'intérêt;

- les moyens de traitement sont adaptés pour mettre en oeuvre un procédé pour estimer un ou plusieurs paramètres hémodynamiques de perfusion (14) selon l'une quelconques des revendications 1 à 17.

19. Unité de traitement comportant des moyens de mémorisation, des moyens pour communiquer avec le monde extérieur et des moyens de traitement, **caractérisée en ce que** :

- les moyens pour communiquer sont aptes à recevoir du monde extérieur des images numériques de perfusion (12, 13) ;
- les moyens de mémorisation comportent un modèle global de perfusion, ledit modèle comportant :

- une première relation entre un signal de perfusion $S(t)$ et une concentration $C(t)$ d'un agent de contraste circulant dans ledit voxel au cours du temps t *;*
- une deuxième relation entre la concentration $C(t)$, le flux sanguin $BF$, un modèle paramétrique ou semi-paramétrique $C_a(t,\Theta_a)$ d'une fonction d'entrée artérielle et un modèle paramétrique ou semi-paramétrique $R(t,\Theta_R)$ d'une fonction de répartition complémentaire du temps de transit dans le voxel, $\Theta_a$ et $\Theta_R$ étant respectivement les paramètres desdits modèles ;
- une troisième relation $\Psi(\Theta_a)=0$ entre les paramètres $\Theta_a$ du modèle de la fonction d'entrée artérielle $C_a(t,\Theta_a)$ consistant à exprimer l'un desdits paramètres en fonction des autres, ladite troisième relation étant identique pour tout voxel d'intérêt et s'exprimant comme: $\int_{0}^{+\infty} C_a(t,\Theta_a)\,dt = C_0$ où $C_0$ est une constante arbitraire non 0 nulle, identique pour tout voxel d'intérêt;

- les moyens de traitement sont adaptés pour déterminer un signal de perfusion $S(t)$ à partir d'images de perfusion (12, 13) reçues et pour mettre en oeuvre un procédé pour estimer un ou plusieurs paramètres hémodynamiques de perfusion (14) selon l'une quelconques des revendications 1 à 17.

20. Unité de traitement selon les revendications 18 ou 19 caractérisé en que les moyens pour communiquer délivrent (52) un ou plusieurs paramètres estimés (14) selon un format approprié à une interface homme-machine (5) apte à restituer à un utilisateur (6) lesdits paramètres estimés.

21. Système d'analyse d'imagerie de perfusion comportant une unité de traitement (4) selon l'une quelconques des revendications 18 à 20 et une interface homme-machine (5) apte à restituer à un utilisateur (6) un ou plusieurs paramètres estimés (14) selon un procédé conforme à l'une quelconque des revendications 1 à 17 et mis en oeuvre par ladite unité de traitement (4).

**Patentansprüche**

1. Verfahren zum Abschätzen von einem oder mehreren hämodynamischen Perfusionsparametern (14) eines elementaren Volumens - eines sogenannten Voxels - eines Organs, wobei das Verfahren durch eine Verarbeitungseinheit (4) eines Analysesystems der Perfusionsbildgebung ausgeführt wird und einen Schritt umfasst, um ausgehend von einem Perfusionssignal $S(t)$ (15) ein oder mehrere hämodynamische Parameter abzuschätzen, wobei der Schritt des Abschätzens des oder der hämodynamischen Parameter(s) darin besteht, eine A-posteriori-Randverteilung (52) für den oder die hämodynamischen Parameter durch die vorherige Zuweisung (51) einer konjugierten A-priori-Verteilung der Parameter $\Theta$ eines globalen Perfusionsmodells auszuwerten, wobei das Modell Folgendes umfasst:

- eine erste Beziehung zwischen dem Perfusionssignal $S(t)$ und einer Konzentration $C(t)$ eines Kontrastmittels, das im Verlauf der Zeit t in diesem Voxel zirkuliert;

- eine zweite Beziehung zwischen der Konzentration $C(t)$ eines Kontrastmittels, dem Blutfluss *BF,* einem parametrischen oder halbparametrischen Modell $C_a(t,\Theta_a)$ einer arteriellen Eintrittsfunktion und einem parametrischen oder halbparametrischen Modell $R(t,\Theta_R)$ einer komplementären kumulativen Verteilungsfunktion der Durchlaufzeit in dem Voxel, wobei $\Theta_a$ und $\Theta_R$ jeweils die Parameter dieser Modelle $C_a(t,\Theta_a)$ und $R(t,\Theta_R)$ sind;

**dadurch gekennzeichnet, dass** dieses globale Perfusionsmodell darüber hinaus eine dritte Beziehung $\Psi(\Theta_a)=0$ zwischen den Parametern $\Theta_a$ des Modells der arteriellen Eintrittsfunktion $C_a(t,\Theta_a)$ umfasst, die darin besteht, einen dieser Parameter als Funktion der anderen auszudrücken, wobei diese dritte Beziehung für jedes interessierende Voxel identisch ist und ausgedrückt wird als $\int_0^{+\infty} C_a\left(t,\Theta_a\right)dt = C_0$ , wobei $C_0$ eine willkürliche Konstante ungleich Null ist, die für jedes interessierende Voxel identisch ist.

2. Verfahren nach Anspruch 1, **dadurch gekennzeichnet dass**:

   - das Perfusionssignal $S(t)$ ausgehend von digitalen Bildern (12, 13), die von einem Perfusionsbildgebungsgerät (1) mittels Kernresonanz geliefert werden, im Voraus erhalten wird,
   - die erste Beziehung des globalen Perfusionsmodells ausgedrückt ist durch $S(t)=S_0 e^{-k \cdot TE \cdot C(t)}$, wobei $S_0$ die mittlere Signalintensität vor der Ankunft des Kontrastmittels in dem Voxel ist, *TE* eine Echozeit ist und *k* eine Konstante ungleich Null ist,
   - die zweite Beziehung des globalen Perfusionsmodells ausgedrückt ist durch $C(t)=\eta.BF \cdot C_a(t,\Theta_a) \otimes R(t,\Theta_R)$, wobei $\otimes$ die Faltung bezeichnet und $\eta$ eine Konstante ungleich Null ist.

3. Verfahren nach Anspruch 1, **dadurch gekennzeichnet dass**:

   - das Perfusionssignal $S(t)$ ausgehend von digitalen Bildern (12, 13), die von einem Perfusionsbildgebungsgerät (1) mittels Computertomographie geliefert werden, im Voraus erhalten wird,
   - die erste Beziehung des globalen Perfusionsmodells eine proportionale Beziehung $S(t)=\alpha.C(t)$ ist, wobei $\alpha$ eine Konstante ungleich Null ist,
   - die zweite Beziehung des globalen Perfusionsmodells ausgedrückt ist durch $C(t)=\eta.BF \cdot C_a(t,\Theta_a) \otimes R(t,\Theta_R)$, wobei $\otimes$ die Faltung bezeichnet und $\eta$ eine Konstante ungleich Null ist.

4. Verfahren nach einem der vorhergehenden Ansprüche, **dadurch gekennzeichnet, dass** es durch sukzessive Iterationen für eine Vielzahl betrachteter Voxels durchgeführt wird.

5. Verfahren nach einem der vorhergehenden Ansprüche, **dadurch gekennzeichnet, dass** die gemeinsame Abschätzung (51) der Parameter $\Theta$ des globalen Perfusionsmodells, die durch die Verarbeitungseinheit (4) erfolgt, mittels des Verfahrens von Bayes oder der Maximum-Likelihood-Methode oder dem Verfahren der kleinsten nichtlinearen Quadrate durchgeführt wird.

6. Verfahren nach einem der vorhergehenden Ansprüche, **dadurch gekennzeichnet, dass** es aus einem Schritt (55) besteht, um die Anpassungsgüte des globalen Perfusionsmodells an die experimentellen Perfusionsdaten $D=[S(t_1),...,S(t_N)]$ durch eine Wahrscheinlichkeitsrechnung $p\left(D\middle|M\right) = \int_\Theta p\left(\Theta\middle|M\right) p\left(D\middle|\Theta,M\right)d\Theta$ dieser Daten unter Kenntnis dieses Modells zu quantifizieren.

7. Verfahren nach einem der vorhergehenden Ansprüche, **dadurch gekennzeichnet, dass** es aus einem vorhergehenden Schritt (56a, 56b, 56c, 57) besteht, um unter einer Vielzahl ein globales Perfusionsmodell auszuwählen.

8. Verfahren nach dem vorhergehenden Anspruch, **dadurch gekennzeichnet, dass** es durch eine Verarbeitungseinheit (4) iterativ für jedes dieser Verarbeitungseinheit bekannte globale Perfusionsmodell angewandt wird.

9. Verfahren nach dem vorhergehenden Anspruch, sofern er vom Anspruch 6 abhängt, **dadurch gekennzeichnet, dass** die Parameter geliefert werden (55a), die nach dem Perfusionsmodell geschätzt wurden, dessen Wahrscheinlichkeit unter Kenntnis dieses Modells am größten ist.

10. Verfahren nach einem der vorhergehenden Ansprüche, **dadurch gekennzeichnet, dass** das Modell $C_a(t,\Theta_a)$ einer arteriellen Eintrittsfunktion ein sogenanntes "Tri-Gamma"-Modell mit zwölf Parametern ist, das folgendermaßen definiert ist:

$$C_a\left(t,\Theta_a\right) = \frac{a\left(t-t_0\right)^{\alpha_0-1} e^{-\left(t-t_0\right)/\beta_0}}{\beta_0^{\alpha_0}\Gamma(\alpha_0)} + \frac{b\left(t-t_1\right)^{\alpha_1-1} e^{-\left(t-t_1\right)/\beta_1}}{\beta_1^{\alpha_1}\Gamma(\alpha_1)} + \frac{c\left(t-t_2\right)^{\alpha_2-1} e^{-\left(t-t_2\right)/\beta_2}}{\beta_2^{\alpha_2}\Gamma(\alpha_2)}$$

$$\Theta_a = \left(a,b,c,\alpha_0,\beta_0,t_0,\alpha_1,\beta_1,t_1,\alpha_2,\beta_2,t_2\right)$$

$$\Gamma(\ ): \quad \text{Eulersche Gammafunktion}$$

11. Verfahren nach einem der vorhergehenden Ansprüche, **dadurch gekennzeichnet, dass** das Modell $R(t,\Theta_R)$ einer komplementären Verteilungsfunktion der Durchgangszeit in das Voxel ein sogenanntes "Korrigiertes Gamma-Integral"-Modell mit zwei Parametern ist, das folgendermaßen definiert ist:

$$R\left(t,\Theta_R\right) = H\left(t\right) - \int_0^t h\left(\tau,MTT,\beta\right)d\tau$$

$$h\left(\tau,MTT,\beta\right) = \frac{\tau^{\frac{MTT}{\beta}-1} e^{-\tau/\beta}}{\beta^{\frac{MTT}{\beta}}\Gamma\left(\frac{MTT}{\beta}\right)} \quad MTT > 0, \beta > 0$$

$$\Theta_R = \left(MTT,\beta\right)$$

$$H(\ ): \quad \text{allgemeine Heaviside-Stufen-Funktion}$$

$$\Gamma(\ ): \quad \text{Eulersche Gammafunktion}$$

12. Verfahren nach einem der vorhergehenden Ansprüche, **dadurch gekennzeichnet, dass** es einen Schritt (53) umfasst, um eine komplementäre Information in Form einer geschätzten arteriellen Eintrittsfunktion $C_a\left(t,\widehat{\Theta_a}\right)$ zu berechnen.

13. Verfahren nach einem der vorhergehenden Ansprüche, **dadurch gekennzeichnet, dass** es einen Schritt (54) umfasst, um eine komplementäre Information in Form einer geschätzten komplementären Verteilungsfunktion $R\left(t,\widehat{\Theta_R}\right)$ zu berechnen.

14. Verfahren nach einem der vorhergehenden Ansprüche, **dadurch gekennzeichnet, dass** es einen Schritt (52a, 53a, 54a) umfasst, um eine komplementäre Information in Form eines mit einem Parameter des globalen Perfusionsmodells zusammenhängenden Konfidenzintervalls zu berechnen.

15. Verfahren nach einem der vorhergehenden Ansprüche, **dadurch gekennzeichnet, dass** es einen Schritt (52b, 53b, 53c) umfasst, um eine komplementäre Information in Form einer mit einem Parameter des globalen Perfusionsmodells zugehörigen Wetterate zu berechnen.

16. Verfahren nach einem der vorhergehenden Ansprüche, **dadurch gekennzeichnet, dass** es einen Schritt des Lieferns (52) von einem oder mehreren geschätzten hämodynamischen Parametern (14) an eine Mensch-Maschine-Schnittstelle (5) umfasst, die einem Nutzer (6) diese geschätzten Parameter wiedergeben kann.

**17.** Verfahren nach einem der Ansprüche 13 bis 16, **dadurch gekennzeichnet, dass** es einen Schritt des Lieferns (52) aller komplementären Informationen an eine Mensch-Maschine-Schnittstelle (5) umfasst, die einem Nutzer (6) die geschätzten Parameter sowie diese komplementären Informationen wiedergeben kann.

**18.** Verarbeitungseinheit (4), umfassend Speichermittel, Mittel zur Kommunikation mit der Außenwelt und Verarbeitungsmittel, **dadurch gekennzeichnet, dass**:

- die Kommunikationsmittel ein Signal $S(t)$ (15) von der Außenwelt empfangen können, das durch die Perfusionsbildgebung erhalten wird;
- die Speichermittel ein globales Perfusionsmodell enthalten, wobei dieses Modell Folgendes umfasst:

- eine erste Beziehung zwischen dem Perfusionssignal $S(t)$ und einer Konzentration $C(t)$ eines Kontrastmittels, das im Verlauf der Zeit $t$ in diesem Voxel zirkuliert;
- eine zweite Beziehung zwischen dem Blutfluss $BF$, der Konzentration $C(t)$ eines Kontrastmittels, einem parametrischen oder halbparametrischen Modell $C_a(t,\Theta_a)$ einer arteriellen Eintrittsfunktion und einem parametrischen oder halbparametrischen Modell $R(t,\Theta_R)$ einer komplementären kumulativen Verteilungsfunktion der Durchlaufzeit in dem Voxel, wobei $\Theta_a$ und $\Theta_R$ jeweils die Parameter dieser Modelle sind;
- eine dritte Beziehung $\psi(\Theta_a)=0$ zwischen den Parametern $\Theta_a$ des Modells der arteriellen Eintrittsfunktion $C_a(t,\Theta_a)$, die darin besteht, einen dieser Parameter als Funktion der anderen auszudrücken, wobei diese dritte Beziehung für jedes interessierende Voxel identisch ist und ausgedrückt wird als

$$\int_{0}^{+\infty} C_a\left(t,\Theta_a\right)dt = C_0,$$ wobei $C_0$ eine willkürliche Konstante ungleich Null ist, die für jedes interessierende Voxel identisch ist;

- die Verarbeitungsmittel angepasst sind, um ein Verfahren nach einem der Ansprüche 1 bis 17 auszuführen, um einen oder mehrere hämodynamische Perfusionsparameter (14) abzuschätzen.

**19.** Verarbeitungseinheit, umfassend Speichermittel, Mittel zur Kommunikation mit der Außenwelt und Verarbeitungsmittel, **dadurch gekennzeichnet, dass**:

- die Kommunikationsmittel digitale Bilder der Perfusion (12, 13) von der Außenwelt empfangen können;
- die Speichermittel ein globales Perfusionsmodell enthalten, wobei dieses Modell Folgendes umfasst:

- eine erste Beziehung zwischen einem Perfusionssignal $S(t)$ und einer Konzentration $C(t)$ eines Kontrastmittels, das im Verlauf der Zeit $t$ in diesem Voxel zirkuliert;
- eine zweite Beziehung zwischen der Konzentration $C(t)$, dem Blutfluss $BF$, einem parametrischen oder halbparametrischen Modell $C_a(t,\Theta_a)$ einer arteriellen Eintrittsfunktion und einem parametrischen oder halbparametrischen Modell $R(t,\Theta_R)$ einer komplementären kumulativen Verteilungsfunktion der Durchlaufzeit in dem Voxel, wobei $\Theta_a$ und $\Theta_R$ jeweils die Parameter dieser Modelle sind;
- eine dritte Beziehung $\psi(\Theta_a)=0$ zwischen den Parametern $\Theta_a$ des Modells der arteriellen Eintrittsfunktion $C_a(t,\Theta_a)$, die darin besteht, einen dieser Parameter als Funktion der anderen auszudrücken, wobei diese dritte Beziehung für jedes interessierende Voxel identisch ist und ausgedrückt wird als

$$\int_{0}^{+\infty} C_a\left(t,\Theta_a\right)dt = C_0,$$ wobei $C_0$ eine willkürliche Konstante ungleich Null ist, die für jedes interessierende Voxel identisch ist;

- die Verarbeitungsmittel angepasst sind, um ein Perfusionssignal $S(t)$ ausgehend von empfangenen Perfusionsbildern (12, 13) zu bestimmen und um ein Verfahren nach einem der Ansprüche 1 bis 17 auszuführen, um einen oder mehrere hämodynamische Perfusionsparameter (14) abzuschätzen.

**20.** Verarbeitungseinheit nach den Ansprüchen 18 oder 19, **dadurch gekennzeichnet, dass** die Kommunikationsmittel, die ein oder mehrere geschätzte Parameter (14) mit einem für eine Mensch-Maschinen-Schnittstelle (5) geeigneten Format liefern (52), wobei die Schnittstelle einem Nutzer (6) diese geschätzten Parameter wiedergegeben kann.

**21.** Analysesystem der Perfusionsbildgebung, umfassend eine Verarbeitungseinheit (4) nach einem der Ansprüche 18 bis 20 und eine Mensch-Maschine-Schnittstelle (5), die einem Nutzer (6) ein oder mehrere geschätzte Parameter wiedergeben kann, die gemäß einem Verfahren nach einem der Ansprüche 1 bis 17 geschätzt (14) wurden, das durch diese Verarbeitungseinheit (4) ausgeführt wird.

**Claims**

**1.** Method for estimating one or more hemodynamic perfusion parameters (14) of an elementary volume - referred to as a voxel - of an organ, said method being implemented by a processing unit (4) of a perfusion imaging analysis system, and comprising a step for estimating one or more hemodynamic parameters from $S(t)$ perfusion signals (15), the step for estimating said hemodynamic parameter(s) consisting in the evaluation of a posterior marginal distribution (52) for said hemodynamic parameter (s) by the prior assignment (51) of a joint a priori distribution of the $\Theta$ parameters of a global perfusion model comprising:

- a first relationship between the perfusion signal $S(t)$ and a concentration $C(t)$ of a contrast agent circulating in the said voxel over time t;
- a second relationship between the concentration $C(t)$ of a contrast agent, the blood flow $BF$, a parametric or semi-parametric model $C_a(t,\Theta_a)$ of an arterial input function and a parametric or semi-parametric model $R(t,\Theta_R)$ of a complementary cumulative distribution function of the transit time in the voxel, $\Theta_a$ and $\Theta_R$ being the parameters of said $C_a(t,\Theta_a)$ and $R(t,\Theta_R)$ models respectively;

**characterized in that** said global perfusion model also comprises a third relationship $\psi(\Theta_a)=0$ between the $\Theta_a$ parameters of the model of the arterial input function $C_a(t,\Theta_a)$ consisting of expressing one of the said parameters as a function of the others, the said third relationship being identical for any voxel of interest and being expressed

as $\displaystyle\int_{0}^{+\infty} C_a\left(t,\Theta_a\right)dt = C_0$ where $C_0$ is a non-zero arbitrary constant, identical for any voxel of interest.

**2.** Method according to claim 1, **characterized in that**:

- the perfusion signal $S(t)$ has been previously obtained from digital images (12, 13) output by a magnetic resonance perfusion imaging device (1);
- the first relationship of the perfusion global model relationship is expressed by $S(t)=S_0e^{-k\cdot TE\cdot C(t)}$ where $S_0$ is the average intensity of the signal before the arrival of the contrast agent in the voxel, $TE$ is an echo time and $k$ is a non-zero constant;
- the second relationship of the perfusion global model is expressed by $C(t)=\eta.BF\cdot C_a(t,\Theta_a)\otimes R(t,\Theta_R)$ where $\otimes$ represents the convolution product and $\eta$ is a non-zero constant.

**3.** Method according to claim 1, **characterized in that**:

- the perfusion signal $S(t)$ has been previously obtained from digital images (12, 13) output by a tomodensitometry perfusion imaging device (1);
- the first relationship of the perfusion global model is a proportionality relationship $S(t)=\alpha.C(t)$ where $\alpha$ is a non-zero constant;
- the second relationship of the perfusion global model is expressed by $C(t)=\eta.BF\cdot C_a(t,\Theta_a)\otimes R(t,\Theta_R)$ where $\otimes$ represents the convolution product and $\eta$ is a non-zero constant.

**4.** Method according to any of the preceding claims, **characterized in that** it is implemented by successive iterations for a plurality of considered voxels.

**5.** Method according to one of the preceding claims, **characterized in that** the joint estimation (51) of the $\Theta$ parameters of the perfusion global model implemented by the processing unit (4) is performed by means of the Bayes method or Maximum Likelihood methods or even non-linear Least Square methods.

**6.** Method according to one of the preceding claims, **characterized in that** it comprises a step (55) for quantifying the goodness-of-fit of the perfusion global model to experimental perfusion data $D=[S(t_1),...,S(t_N)]$ by a calculation of a

probability $p\big(D\big|M\big)=\int_{\Theta}p\big(\Theta\big|M\big)p\big(D\big|\Theta,M\big)d\Theta$ of these data knowing said model.

**7.** Method according to any of the preceding claims, **characterized in that** it comprises a prior step (56a, 56b, 56c, 57) in order to choose one from a plurality of perfusion global models.

**8.** Method according to the preceding claim, **characterized in that** it is implemented iteratively by the processing unit (4) for each known perfusion global model of said processing unit.

**9.** Method according to the preceding claim when it depends on claim 6, **characterized in that** the estimated parameters (55a) are output according to the perfusion model whose probability knowing the said model is highest.

**10.** Method according to one of the preceding claims, **characterized in that** the $C_a(t,\Theta_a)$ model of an arterial input function is a so-called "tri-Gamma" model with twelve parameters defined by:

$$\begin{cases} C_a\left(t,\Theta_a\right)=\dfrac{a\left(t-t_0\right)^{\alpha_0-1}e^{-\left(t-t_0\right)/\beta_0}}{\beta_0^{\alpha_0}\Gamma(\alpha_0)}+\dfrac{b\left(t-t_1\right)^{\alpha_1-1}e^{-\left(t-t_1\right)/\beta_1}}{\beta_1^{\alpha_1}\Gamma(\alpha_1)}+\dfrac{c\left(t-t_2\right)^{\alpha_2-1}e^{-\left(t-t_2\right)/\beta_2}}{\beta_2^{\alpha_2}\Gamma(\alpha_2)} \\[2mm] \Theta_a=\left(a,b,c,\alpha_0,\beta_0,t_0,\alpha_1,\beta_1,t_1,\alpha_2,\beta_2,t_2\right) \\[2mm] \Gamma(\ )\ :\ \text{Euler Gamma function} \end{cases}$$

**11.** Method according to one of the preceding claims, **characterized in that** the model $R(t,\Theta_R)$ of a complementary cumulative distribution function of the transit time in the voxel is a so-called "Corrected Integral Gamma" model with two parameters defined by:

$$\begin{cases} R\left(t,\Theta_R\right)=H\left(t\right)-\int_0^t h\left(\tau,MTT,\beta\right)d\tau \\[2mm] h\left(\tau,MTT,\beta\right)=\dfrac{\tau^{\frac{MTT}{\beta}-1}e^{-\tau/\beta}}{\beta^{\frac{MTT}{\beta}}\Gamma\left(\dfrac{MTT}{\beta}\right)} \quad MTT>0,\beta>0 \\[2mm] \Theta_R=(MTT,\beta) \\[2mm] H(\ )\ :\ \text{Heaviside generalized step function} \\[2mm] \Gamma(\ )\ :\ \text{Euler Gamma function} \end{cases}$$

**12.** Method according to any one of the preceding claims, **characterized in that** it comprises a step (53) to calculate additional information in the form of an estimated arterial input function $C_a\left(t,\widehat{\Theta_a}\right)$.

**13.** Method according to any one of the preceding claims, **characterized in that** it comprises a step (54) to calculate additional information in the form of an estimated complementary cumulative distribution function $R\left(t,\widehat{\Theta_R}\right)$.

**14.** Method according to any one of the preceding claims, **characterized in that** it comprises a step (52a, 53a, 54a) to calculate additional information in the form of a confidence interval associated with a parameter of the perfusion global model.

15. Method according to any one of the preceding claims, **characterized in that** it comprises a step (52b, 53b, 53c) to calculate additional information in the form of a bet rate associated with a parameter of the perfusion global model.

16. Method according to any of the preceding claims, **characterized in that** it comprises a step (52) to deliver one or more estimated hemodynamic parameters (14) at a man/machine interface (5) capable of outputting to a user (6) the said estimated parameters.

17. Method according to any one of claims 13 to 16, **characterized in that** it comprises a step (52) for delivering any additional information to a man/machine interface (5) capable of outputting to a user (6) the estimated parameters as well as the said additional information.

18. Processing unit (4) comprising storage means, means for communicating with the outside world and processing means, **characterized in that**:

- the communication means are capable of receiving from the outside world a signal $S(t)$ (15) obtained by perfusion imaging;
- the storage means comprise a perfusion global model, said model comprising:

- a first relationship between the perfusion signal $S(t)$ and a concentration $C(t)$ of a contrast agent circulating in the said voxel over time $t$;
- a second relationship between the blood flow $BF$ in the voxel, the concentration $C(t)$, a parametric or semi-parametric model $C_a(t,\Theta_a)$ of an arterial input function and a parametric or semi-parametric model $R(t,\Theta_R)$ of a complementary cumulative distribution function of the transit time in the voxel, $\Theta_a$ and $\Theta_R$ being the parameters of said models respectively;
- a third relationship $\psi(\Theta_a)=0$ between the $\Theta_a$ parameters of the model of the arterial input function $C_a(t,\Theta_a)$ consisting in expressing one of the said parameters as a function of the others, the said third relationship being identical for any voxel of interest and being expressed as $\int_{0}^{+\infty} C_a(t,\Theta_a)\,dt = C_0$ where $C_0$ is a non-zero arbitrary constant, identical for any voxel of interest;

- the processing means are adapted to implement a method for estimating one or more perfusion hemodynamic parameters (14) according to any one of claims 1 to 17.

19. Processing unit comprising storage means, means to communicate with the outside world and processing means, **characterized in that**:

- the communication means are capable of receiving from the outside world perfusion digital images (12, 13);
- the storage means comprise a perfusion global model, said model comprising:

- a first relationship between the perfusion signal $S(t)$ and a concentration $C(t)$ of a contrast agent circulating in the said voxel over time $t$;
- a second relationship between the concentration $C(t)$, the blood flow $BF$, a parametric or semi-parametric model $C_a(t,\Theta_a)$ of an arterial input function and a parametric or semi-parametric model $R(t,\Theta_R)$ of a complementary cumulative distribution function of the transit time in the voxel, $\Theta_a$ and $\Theta_R$ being the parameters of said models respectively;
- a third relationship $\psi(\Theta_a)=0$ between the $\Theta_a$ parameters of the model of the arterial input function $C_a(t,\Theta_a)$ consisting in expressing one of the said parameters as a function of the others, the said third relationship being identical for any voxel of interest and being expressed as $\int_{0}^{+\infty} C_a(t,\Theta_a)\,dt = C_0$ where $C_0$ is a non-zero arbitrary constant, identical for any voxel of interest;

- the processing means are adapted to determine a perfusion signal $S(t)$ from perfusion images (12, 13) received and to implement a method for estimating one or more perfusion hemodynamic parameters (14) according to any of claims 1 to 17.

20. Processing unit according to claims 18 or 19, **characterized in that** the communication means deliver (52) one or more estimated parameters (14) in a format appropriate for a man/machine interface (5) capable of outputting to a user (6) the said estimated parameters.

21. Perfusion imaging analysis system comprising a processing unit (4) according to any of claims 18 to 20 and a man/machine interface (5) capable of outputting to a user (6) one or more estimated parameters (14) adopting a method according to any of claims 1 to 17 and implemented by the said processing unit (4).

FIG.1

FIG.2

FIG.3

FIG.4

**FIG.5a**

**FIG.5b**

**FIG.6**

**FIG.7**

**FIG.8**

FIG.9

FIG.10

FIG.11

FIG.12

FIG.13

FIG.14

EP 2 437 664 B1

**FIG.15**  81

82  **FIG.16**

**FIG.17**

33

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **VONKEN.** *Maximum Likelihood Estimation of Cerebral Blood Flow in Dynamic Susceptibiliy Contrat MRI*
- **MOURIDSEN.** *Bayesian estimation of cerebral perfusion using a physiological model of micro vasculature*
- **KIM.** *Multiphasic contrast injection for improved precision of parameter estimated in functional CT*
- **BRUNECKER.** *Correcting saturation effects of the arterial input function in Dynamic Susceptibility Contrast-enhanced MRI*
- **SHIMONY.** *Estimation of Cerebral Blood Flow from Susceptibility Contrast MRI Using a Tissue Model*